# EUROPÄISCHE PATENTANMELDUNG

(11) **EP 2 384 752 A1**
(43) Veröffentlichungstag der Anmeldung: **09.11.2011**
(21) Anmeldenummer: 10003953.6
(22) Anmeldetag: 14.04.2010
(51) Int. Cl.: A61K 31/405, A61K 31/519, A61K 31/522, A61K 31/5415, A61K 45/06, A61P 35/00

(54) **Kombinationspräparat umfassend einen Phosphodiesterasehemmer und einen COX-Inhibitor zur Behandlung von Krebs**

(71) Anmelder: Universitätsklinikum Hamburg-Eppendorf, 20246 Hamburg (DE)
(72) Erfinder: Schumacher, Udo, 21465 Wentorf (DE)
(74) Vertreter: UEXKÜLL & STOLBERG

(57) **Zusammenfassung**

Die vorliegende Erfindung betrifft pharmazeutische Zusammensetzungen, die mindestens einen Phosphodiesterasehemmer und einen Cyclooxygenase (COX)-Inhibitor umfassen, insbesondere für die Behandlung von Krebserkrankungen. Die Erfindung betrifft ferner die Verwendung eines Phosphodiesterasehemmers oder eines COX-Inhibitors zur Herstellung einer pharmazeutischen Zusammensetzung für die Behandlung einer Krebserkrankung, wobei die Zusammensetzung mindestens einen Phosphodiesterasehemmer und mindestens einen COX-Inhibitor umfasst. Die Erfindung stellt ferner Kits bereit, die mindestens einen Phosphodiesterasehemmer und mindestens einen COX-Inhibitor umfassen.

## Beschreibung

Die vorliegende Erfindung betrifft pharmazeutische Zusammensetzungen, die mindestens einen Phosphodiesterasehemmer und einen Cyclooxygenase (COX)-Inhibitor umfassen, insbesondere für die Behandlung von Krebserkrankungen. Die Erfindung betrifft ferner die Verwendung eines Phosphodiesterasehemmers oder eines COX-Inhibitors zur Herstellung einer pharmazeutischen Zusammensetzung für die Behandlung einer Krebserkrankung, wobei die Zusammensetzung mindestens einen Phosphodiesterasehemmer und mindestens einen COX-Inhibitor umfasst. Die Erfindung stellt ferner Kits bereit, die mindestens einen Phosphodiesterasehemmer und mindestens einen COX-Inhibitor umfassen.

### HINTERGRUND DER ERFINDUNG

Neoplastische Erkrankungen zeichnen sich durch ein unkontrolliertes Wachstum anormaler Zellen aus, wobei sich diese Zellen unkontrolliert vermehren und Metastasen in anderen Organen bilden können. In den Industriestaaten lassen sich etwa 20% aller Todesfälle auf Krebserkrankungen zurückführen.

Die gegenwärtig in der klinischen Therapie eingesetzten Konzept stützen sich im Wesentlichen auf die chirurgische Entfernung des neoplastischen Gewebes, die Strahlentherapie und die Behandlung mit Chemotherapeutika. Eine Chemotherapie wird in der Regel eingesetzt, wenn die Erkrankung verhältnismäßig weit fortgeschritten ist. Ferner kann die Behandlung mit Chemotherapeutika im Falle von primär generalisierten Erkrankungen, wie z.B. den hämatologischen Krebserkrankungen indiziert sein. Die Behandlung mit Chemotherapeutika beruht auf der gegenüber somatischen Zellen gesteigerten Proliferationsrate von Krebszellen, die mit einer erhöhten Empfindlichkeit gegenüber cytotoxischen Substanzen einhergeht. Chemotherapeutika, die sich für die Behandlung bestimmter neoplastischer Erkrankungen eignen, sind im Stand der Technik in großer Zahl beschrieben worden. Einen Überblick bietet die folgende Publikation (De Vita, Hellman & Rosenberg, Cancer: Principles and Practice of Oncology, 7. Auflage 2004, Lipincott, Williams & Wilkins).

Allerdings sprechen etwa die Hälfte aller neoplastischen Gewebe nicht oder nur unzureichend auf eine Behandlung mit den gegenwärtig verfügbaren Cytostatika an. Dies gilt insbesondere für Tumore, die sich von Lunge, Kolon, Pankreas, Leber und Niere ableiten. Diese Tumore sind im allgemeinen gegen die heute zur Verfügung stehenden Chemotherapeutika unempfindlich, da sie über Mechanismen verfügen, die eine Resistenz gegen diese Mittel bewirken. Ferner ist bereits frühzeitig in der klinischen Praxis bemerkt worden, dass viele neoplastische Gewebe, die zunächst empfindlich gegenüber den eingesetzten chemotherapeutischen Mitteln sind, im weiteren Behandlungsverlauf diese Empfindlichkeit verlieren und zunehmend resistent werden. Dies hat zur Folge, dass sich die behandelten Tumoren nach mehreren Chemotherapiezyklen hinsichtlich ihres Wachstums nicht mehr beeinflussen lassen. Aus diesem Grunde ist es wichtig, weitere Wirkstoffe oder Wirkstoffkombinationen zu identifizieren und bereitzustellen, die für die Behandlung solcher resistenten Tumore eingesetzt werden können.

Vor diesem Hintergrund liegt der vorliegenden Erfindung somit die Aufgabe zugrunde, neue pharmazeutische Zusammensetzungen und Präparate bereitzustellen, mit deren Hilfe eine effektive Behandlung verschiedenartiger Krebserkrankungen möglich ist. Erfindungsgemäß wird diese Aufgabe durch die pharmazeutischen Zusammensetzungen und Präparate gemäß den anliegenden Ansprüchen gelöst.

Im Rahmen der vorliegenden Erfindung wurde nunmehr überraschend herausgefunden, dass Wirkstoffkombinationen aus mindestens einem Phosphodiesterasehemmer und mindestens einem Cyclooxygenase (COX)-Inhibitor eine starke antiproliferative Wirkung auf verschiedene neoplastische Zellen ausüben. Die vorliegende Erfindung stellt demgemäß in einem ersten Aspekt eine pharmazeutische Zusammensetzung bereit, welche die folgenden Komponenten umfasst:
a) mindestens einen Phosphodiesterasehemmer, und
b) mindestens einen COX-Inhibitor.

Die beiden Wirkstoffe können Teil derselben pharmazeutischen Formulierung sein. Dies bedeutet, dass der Phosphodiesterasehemmer gemeinsam mit dem COX-Inhibitor als einheitliche Formulierung, d.h. als einheitliche pharmazeutische Zusammensetzung, verabreicht werden kann, z.B. in Form einer Tablette für die orale Verabreichung. Die erfindungsgemäße pharmazeutische Zusammensetzung eignet sich insbesondere für die Behandlung einer Krebserkrankung. Es ist allerdings für die Ausführung der vorliegenden Erfindung nicht zwingend notwendig, dass die oben aufgeführten Komponenten in einer einheitlichen pharmazeutischen Zusammensetzung vorliegen. Vielmehr können der oder die Phosphodiesterasehemmer und der oder die COX-Inhibitor(en) auch in getrennten Formulierungen vorliegen, die gleichzeitig oder zeitlich versetzt an den zu behandelnden Patienten verabreicht werden.

Somit betrifft die Erfindung in einem zweiten Aspekt ein pharmazeutisches Kombinationspräparat, umfassend:
a) mindestens einen Phosphodiesterasehemmer, und
b) mindestens einen COX-Inhibitor,
für die gleichzeitige oder zeitlich getrennte Anwendung in der Therapie, d.h. bei der therapeutischen Behandlung einer Erkrankung insbesondere einer Krebserkrankung.

Die beiden Wirkstoffe der erfindungsgemäßen Kombination können beispielsweise ex vivo, d.h. vor der Verabreichung an den Patienten, miteinander zu einer wie oben beschriebenen einheitlichen Darreichungsform kombiniert werden. So lassen sich beispielsweise ein oder mehrere Phosphodiesterasehemmer, die gemeinsam mit einem oder mit mehreren COX-Inhibitoren in einer lyophilisierten Mischung vorliegen, durch Zugabe eines geeigneten Lösungsmittels zu einer Infusionslösung oder Injektionslösung rekonstituieren, die beide Wirkstoffgruppen umfasst.

Alternativ dazu können der oder die Phosphodiesterasehemmer und der oder die COX-Inhibitor(en) in getrennten Formulierungen vorliegen, die zum selben Zeitpunkt oder zu unterschiedlichen Zeitpunkten an den Patienten verabreicht werden. So können beispielsweise Phosphodiesterasehemmer und COX-Inhibitor an verschiedenen Tagen eines Behandlungszyklus verabreicht werden. Der Phosphodiesterasehemmer kann beispielsweise im Rahmen eines sich wiederholenden einwöchigen Behandlungszyklus täglich verabreicht werden, wobei der COX-Inhibitor lediglich an einem bestimmten Tag dieses Zyklus verabreicht wird. Sofern der (oder die) Phosphodiesterasehemmer und der (oder die) COX-Inhibitor(en) zeitlich getrennt voneinander verabreicht werden sollen, ist es zweckmäßig, die beiden Wirkstoffe in getrennten Verpackungseinheiten (z.B. in mehreren Ampullen) bereitzustellen. Dabei können der (oder die) Phosphodiesterasehemmer und der (oder die) COX-Inhibitor(en) in gleichartigen oder unterschiedlichen Darreichungsformen vorliegen, die nachstehend genauer beschrieben werden.

Aufgrund der antiproliferativen Wirkung der erfindungsgemäßen Kombination aus Phosphodiesterasehemmer und COX-Inhibitor lassen sich die pharmazeutischen Zusammensetzungen und Präparate der vorliegenden Erfindung in zweckmäßiger Weise zur Behandlung von verschiedenen Krebserkrankungen einsetzen. Dabei sind die Zusammensetzungen und Präparate hinsichtlich ihrer Anwendung nicht auf bestimmte Krebsarten beschränkt. Krebserkrankungen, die wirksam durch die erfindungsgemäßen Zusammensetzungen und Präparate behandelt werden können, umfassen z.B. Bronchialkarzinome (kleinzelliges Bronchialkarzinom und großzelliges Bronchialkarzinom, Kolonkrebs, Brustkrebs, Prostatakrebs, Leberkrebs, Pankreaskrebs, Blasenkrebs, Hautkrebs (z.B. Melanome), Eierstockkrebs, hämatologische Krebserkrankungen, Krebserkrankungen des Gehirns, Magenkrebs, Nierenkrebs, Uteruskrebs, Knochenkrebs, Ösophaguskrebs, Kaposi-Sarkom, oropharyngale Krebserkrankungen, Hodenkrebs, Schilddrüsenkrebs, Lymphome, adenokortikale Krebserkrankungen, Gallenblasenkrebs, multiples Myelom, Dünndarmkrebs, Analkrebs, Bauchspeicheldrüsenkrebs, Burkitt-Lymphom, Gallengangkrebs, Gebärmutterhalskrebs, Gebärmutterkörperkrebs, Harnröhrenkrebs, Kehlkopfkrebs, Morbus Hodgkin, Non-Hodgkin-Lymphom, Wilms-Tumor, Plasmocytom oder Retionblastom.

Gemäß einer bevorzugten Ausführungsform der vorliegenden Erfindung ist die zu behandelnde Krebserkrankung ein Bronchialkarzinom. Dabei handelt es sich um eine Neoplasie, die von den Schleimhautzellen ausgeht, welche das Innere der Bronchien oder Bronchiolen auskleiden. Das Bronchialkarzinom macht über 95% aller bösartigen Tumoren der Lunge aus. Symptome des Bronchialkarzinoms sind häufig anhaltender Husten, Atemnot und Schmerzen im Brustkorb. Zu den Bronchialkarzinomen zählen das Plattenepithelkarzinom, das Adenokarzinom, das großzellige Bronchialkarzinom sowie das kleinzellige Bronchialkarzinom. Die Bronchialkarzinome unterscheiden sich hinsichtlich ihrer histologischen Eigenschaften insbesondere durch ihr Wachstums- und Ausbreitungsverhalten.

Gemäß einer besonders bevorzugten Ausführungsform der vorliegenden Erfindung handelt es sich bei der zu behandelnden Krebserkrankung um das kleinzellige Bronchialkarzinom. Diese Art von Bronchialkarzinom macht ungefähr 20-25% aller diagnostizierten Bronchialkarzinome aus. Das kleinzellige Bronchialkarzinom geht von den neuroendokrinen APUD-Zellen aus und siedelt sich meist zentral in der Lunge an. Kleinzellige Bronchialkarzinome stellen eine besonders aggressive Krebsart dar, da ihre frühe lymphogene und hämatogene Metastasierung dazu führt, dass sich in der Regel schon vor der Entdeckung des eigentlichen Karzinoms Metastasen gebildet haben. Das kleinzellige Lungenkarzinom wächst sehr schnell und breitet sich rasch über Blut- und Lymphgefäße in Lunge, Skelett, Knochenmark, Leber und Gehirn aus. Das kleinzellige Bronchialkarzinom ist praktisch inoperabel. Auch die derzeit verfügbaren Therapieansätze durch Bestrahlung und/oder Chemotherapie führen nicht zu wesentlichen Erfolgen. In der Regel führt das kleinzellige Bronchialkarzinom bei praktisch allen Patienten innerhalb von zwei Jahren nach Stellung der Diagnose zum Tod. Die nicht-kleinzelligen Bronchialkarzinome, zum Beispiel das Plattenepithelkarzinom, das Adenokarzinom und das großzellige Bronchialkarzinom, wachsen und metastasieren im Vergleich zum kleinzelligen Lungenkarzinom wesentlich langsamer und sind daher mit höheren Therapieerfolgen assoziiert.

Erfindungsgemäß umfassen die bereitgestellten pharmazeutischen. Zusammensetzungen und Präparate mindestens einen Phosphodiesterasehemmer. Wie vorliegend verwendet, bezeichnet der Begriff Phosphodiesterasehemmer einen pharmazeutischen Wirkstoff, der zu einer (vollständigen oder teilweisen) Hemmung eines Phosphodiesterase-Enzyms, vorzugsweise eines humanen Phosphodiesterase-Enzyms, führt. Als Phosphodiesterasen werden vorliegend Enzyme in Vertebraten bezeichnet, welche den Abbau der Botensubstanzen cAMP und cGMP zu AMP bzw. GMP katalysieren. Phosphodiesterasen lassen sich in verschiedene Subtypen klassifizieren. Inhibitoren von Phosphoesterasen (Phosphodiesterasehemmer) können mit Hilfe kommerziell erhältlicher Kits identifiziert werden. Es können zu diesem Zweck z.B. der PDE-Glo™ Phosphodiesterase Assay (Promega) oder der Phosphodiesterase [3H]cGMP SPA Enzyme Assay (GE Healthcare) eingesetzt werden. Erfindungsgemäß können verschiedene kommerziell erhältliche Phosphodiesterasehemmer zur Herstellung der pharmazeutischen Zusammensetzungen und Präparate verwendet werden. Solche Phosphodiesterasehemmer umfassen insbesondere 1-Methyl-3-isobutylxanthin (IBMX), Theophyllin, CC-8062, CC-8075, Vinpocetin, Anagrelid, EHNA (Erythro-9-(2-hydroxy-3-nonyl)adenin), Milrinon, Enoximon, Mesembrin, Rolipram, Ibudilast, Piclamilast, Pentoxyfyllin, Sildenafil, Tadalafil, Vardenafil, Udenafil, Avanafil, Dipyridamol oder pharmazeutisch geeignete Salze oder Solvate davon. Der Fachmann wird erkennen, dass auch Derivate der oben genannten Phosphodiesterasehemmer verwendet werden können, solange die gewünschte pharmakologische Aktivität erhalten bleibt.

Gemäß einer bevorzugten Ausführungsform ist der Phosphodiesterasehemmer, der im Rahmen der pharmazeutischen Zusammensetzung und Präparate verwendet wird, ein Phosphodiesterasehemmer, von dem bekannt ist, dass der bereits bei alleiniger Gabe eine cytostatische und/oder cytotoxische Wirkung auf Krebszellen ausübt. Eine solche Wirkung wurde beispielsweise für die Phosphodiesterasehemmer 1-Methyl-3-isobutylxanthin (IBMX), CC-8062, CC-8075, Pentoxyfylline beschrieben. (Mouratidis et al. (2009) Pancreas, 38(1):78-84; Dua und Gude (2006) Cancer Chemother. Pharmacol., 58(2):195-202).

Gemäß einer besonders bevorzugten Ausführungsform handelt es sich bei dem in der pharmazeutischen Zusammensetzung zu verwendenden Phosphodiesterasehemmer um 1-Methyl-3-isobutylxanthin oder um ein pharmazeutisch geeignetes Derivat, Salz oder Solvat davon. Die IUPAC Bezeichnung für 1-Methyl-3-isobutylxanthin lautet 1-Methyl-3-(2-methylpropyl)-7H-purine-2,6-dione. 1-Methyl-3-isobutylxanthin weist die folgende Strukturformel auf:

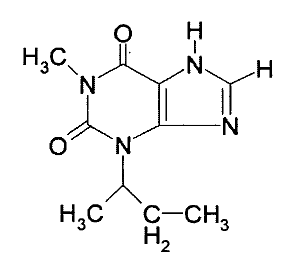

Das 1-Methyl-3-isobutylxanthin oder das pharmazeutisch geeignete Derivat, Salz oder Solvat davon wird vorzugsweise in Kombination mit Indometacin und/oder Meloxicam oder einem pharmazeutisch geeigneten Derivat, Salz oder Solvat dieser Verbindungen verwendet (siehe unten). Das 1-Methyl-3-isobutylxanthin kann mit einem der nachstehend beschriebenen Cyclooxygenase (COX)-Inhibitoren kombiniert werden.

Gemäß einer alternativen Ausführungsform handelt es sich bei dem in der pharmazeutischen Zusammensetzung zu verwendenden Phosphodiesterasehemmer um Sildenafil oder um ein pharmazeutisch geeignetes Derivat, Salz oder Solvat davon. Das Sildenafil kann mit einem beliebigen der nachstehend beschriebenen Cyclooxygenase (COX)-Inhibitoren kombiniert werden. Der Wirkstoff Sildenafil wird von der Firma Pfizer unter der Bezeichnung Viagra^{©} zur Behandlung der erektilen Dysfunktion vertrieben. Die IUPAC Bezeichnung von Sildenafil lautet 1-{[3-(1-Methyl-7-oxo-3-propyl-6,7-dihydro-1H-pyrazolo[4,3-d]pyrimidin-5-yl)-4-ethoxyphenyl]sulfonyl}-4-methyl-piperazin. Sildenafil weist die folgende Strukturformel auf:

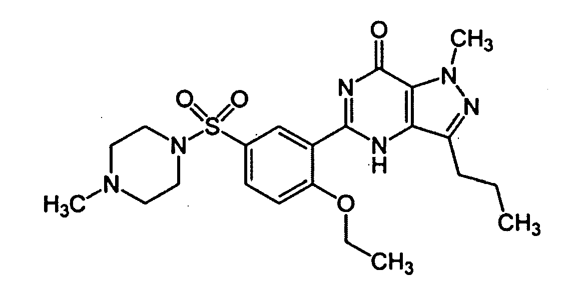

Dem Fachmann ist ersichtlich, dass die oben dargestellten Strukturen von 1-Methyl-3-isobutylxanthin und Sildenafil an einer oder an mehreren Stellen substituiert oder anderweitig modifiziert werden können, solange diese Veränderungen die antiproliferative Wirkung von 1-Methyl-3-isobutylxanthin bzw. Sildenafil, die in Kombination mit dem COX-Inhibitor (z.B. Indometacin oder Meloxicam) beobachtet wird, nicht wesentlich beeinträchtigen werden und unter toxischen Gesichtspunkten nicht zu nachteiligen Ergebnissen führen. Beispielsweise können ein oder mehrere Wasserstoffatome der C-H bzw. N-H Bindungen durch Halogenatome, wie beispielsweise Chlor-, Brom- oder Iod-Atome, substituiert sein. Ferner kann der Wasserstoff der C-H Bindungen auch gegen eine Alkylgruppe, wie beispielsweise Methyl, Ethyl oder Propyl ersetzt werden.

Die erfindungsgemäß bereitgestellten pharmazeutischen Zusammensetzungen und Präparate umfassen neben einem oder mehreren Phosphodiesterasehemmern auch einen oder mehrere Cyclooxygenase (COX)-Inhibitoren oder pharmazeutisch geeignete Derivate, Salze oder Solvate derselben. Wie vorliegend verwendet, bezeichnet der Begriff Cyclooxygenase-Inhibitor oder COX-Inhibitor einen pharmazeutischen Wirkstoff, der zu einer (vollständigen oder teilweisen) Hemmung eines Cyclooxygenase-Enzyms, vorzugsweise eines humanen Cyclooxygenase-Enzyms führt. Der Inhibitor ist vorzugsweise ein COX-2-Inhibitor. Verfahren zur Identifizierung von Inhibitoren der humanen Cyclooxygenase sind hinreichend bekannt und wurden in der Literatur ausführlich beschrieben (siehe, z.B. Griese & Kobolt, Current Protocols in Pharmacology, Unit Number: UNIT 3.1 DOI: 10.1002/0471141755.ph0301s00, Online Posting Date: May, 2001, oder die entsprechende Printversion). Eine ausführliche Darstellung zu COX-2 Inhibitoren ist in WO 02/062391 zu finden. Erfindungsgemäß können verschiedene kommerziell erhältliche COX-Inhibitoren zur Herstellung der pharmazeutischen Zusammensetzungen und Präparate verwendet werden. Solche COX-Inhibitoren umfassen insbesondere Indometacin, Meloxicam, Valdecoxib, Celecoxib, Rofecoxib, Aspirin, Ibuprofen, NS-398, Nimesulid oder pharmazeutisch geeignete Salze oder Solvate davon. Der Fachmann wird erkennen, dass auch Derivate der genannten COX-Inhibitoren verwendet werden können, solange die gewünschte pharmakologische Aktivität, d.h. der cytostatische und/oder cytotoxische Effekt, welche in Kombination mit einem oder mit mehreren Phosphodiesterasehemmern beobachtet wird, erhalten bleibt oder zumindest nicht im erheblichen Ausmaß eingeschränkt wird.

Besonders bevorzugt sind solche COX-Inhibitoren, von denen bekannt ist, dass sie bereits bei alleiniger Gabe eine cytostatische und/oder cytotoxische Wirkung auf Krebszellen ausüben. Eine solche Wirkung ist beispielsweise für die COX-Inhibitoren Indometacin, Celecoxib, NS-398 und Nimesulid beschrieben. Gemäß einer besonders bevorzugten Ausführungsform handelt es sich bei dem in der pharmazeutischen Zusammensetzung/Präparat zu verwendenden COX-Inhibitor um Indometacin oder um ein pharmazeutisch geeignetes Derivat, Salz oder Solvat von Indometacin. Die offizielle IUPAC Bezeichnung für Indometacin ist 2-[1-(4-Chlorbenzoyl)-5-methoxy-2-methyl-1H-indol-3-yl]essigsäure. Indometacin weist die folgende Strukturformel auf:

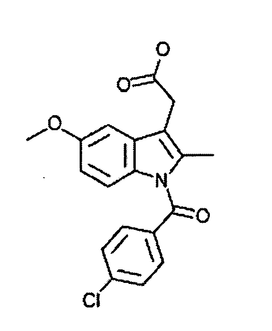

Gemäß einer weiteren Ausführungsform handelt es sich bei dem in der pharmazeutischen Zusammensetzung/Präparat zu verwendenden COX-Inhibitor um Meloxicam oder um ein pharmazeutisch geeignetes Derivat, Salz oder Solvat von Meloxicam. Die offizielle IUPAC Bezeichnung für Meloxicam ist 4-Hydroxy-2-methyl-N-(5-methyl-2-thiazolyl)-2H-1,2-benzothiazin-3-carboxamid-1,1-dioxid. Meloxicam weist die folgende Strukturformel auf:

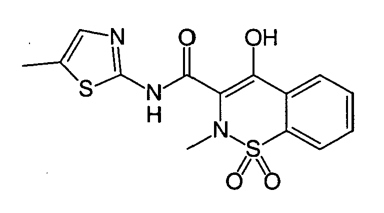

Der Fachmann wird erkennen, dass die oben dargestellten Strukturen von Indometacin und Meloxicam an einer oder an mehreren Stellen substituiert oder anderweitig modifiziert sein können, solange diese Veränderungen die antiproliferative Wirkung der Substanz, die in Kombination mit einem Phosphodiesterasehemmer wie IBMX oder Sildenafil beobachtet wird, nicht wesentlich beeinträchtigen und auch keine für den Patienten nicht tolerierbare toxische Nebenwirkungen nach sich ziehen. Beispielsweise können ein oder mehrere Wasserstoffatome der C-H Bindungen des heterocyclischen Ringsystems mit einem Halogenatom, wie beispielsweise einem Chlor-, Brom- oder Iod-Atom, substituiert sein. Ferner kann der Wasserstoff dieser C-H Bindungen oder der N-H-Bindungen auch gegen eine Alkylgruppe, wie beispielsweise Methyl, Ethyl oder Propyl substituiert sein.

In einer besonders bevorzugten Ausführungsform umfasst die erfindungsgemäße Zusammensetzung oder das erfindungsgemäße Präparat den Phosphodiesterasehemmer 1-Methyl-3-isobutylxanthin (IBMX) oder ein pharmazeutisch geeignetes Derivat, Salz oder Solvat desselben in Kombination mit dem COX-Inhibitor Indometacin und/oder Meloxicam bzw. einem pharmazeutisch geeigneten Salz oder Solvat desselben. Alternativ dazu umfasst die erfindungsgemäße Zusammensetzung oder das erfindungsgemäße Präparat den Phosphodiesterasehemmer Sildenafil oder ein pharmazeutisch geeignetes Derivat, Salz oder Solvat desselben in Kombination mit dem COX-Inhibitor Indometacin und/oder Meloxicam bzw. einem pharmazeutisch geeigneten Derivat, Salz oder Solvat desselben.

Wie vorliegend verwendet bezeichnen Derivate der oben genannten Verbindung chemisch oder biologisch modifizierte Versionen der Ausgangsverbindung, die sich von diesen in struktureller Hinsicht geringfügig unterscheiden. Wie oben erwähnt, können die Derivate z.B. im Vergleich zu den Ausgangsverbindungen an einer oder an mehreren Stellen substituiert oder anderweitig modifiziert sein. So können ein oder mehrere Wasserstoffatome von C-H Bindungen eines heterocyclischen Ringsystems z.B. gegen ein Halogenatom, z.B. Chlor, Brom oder Iod, ausgetauscht sein. Ferner kann Wasserstoff einer C-H Bindung oder einer N-H-Bindung gegen eine Alkylgruppe, wie z.B. Methyl, Ethyl oder Propyl, ausgetauscht sein. Diese Modifikationen stellen jeweils Derivate im Sinne der Erfindung dar. Derivate umfassen darüber hinaus beispielsweise Ester, Ether, Amide sowie tautomere und stereoisomere Formen der genannten Verbindungen. Das Derivat kann eine bestimmte Eigenschaft oder Aktivität des Ausgangsmoleküls in verstärktem oder vermindertem Umfang aufweisen. Es ist jedoch auch möglich, dass das Derivat eine bestimmte Eigenschaft oder Aktivität des Ausgangsmoleküls nicht aufweist, oder gegenüber dem Ausgangsmolekül, von dem es abgeleitet wurde, neue Eigenschaften oder Aktivitäten zeigt.

Ein pharmazeutisch geeignetes Salz umfasst insbesondere nicht-toxische, SäureAdditionssalze sowie Alkalimetall- bzw. Erdalkalimetallsalze. Beispielhafte Säure-Additionssalze umfassen Hydrochlorid, Hydrobromid, Sulfat, Bisulfat, Acetat, Oxalat, Phosphat, Citrat, Maleat, Fumarat, Succinat, Tartrat und Laurylsulfat. Beispielhafte Alkalimetall- bzw. Erdalkalimetallsalze umfassen Natrium-, Kalium-, Calcium- und Magnesium-Salze. Darüber hinaus können erfindungsgemäß auch Ammoniumsalze und Salze mit organischen Aminen verwendet werden.

Solvate der oben genannten Verbindungen sind ebenfalls Teil der vorliegenden Erfindung. Solvate entstehen durch die Anlagerung von einem oder mehreren Lösungsmittelmolekülen an eine erfindungsgemäß vorgeschlagene Wirkstoffverbindung (d.h. an den Phosphodiesterasehemmer oder den COX-Inhibitor). Ist das Lösungsmittel Wasser, so handelt es sich um eine Hydratation. Solvate der vorgeschlagenen Wirkstoffverbindung können durch Ionenbindungen und/oder kovalente Bindungen zusammengehalten werden.

Die erfindungsgemäßen pharmazeutischen Zusammensetzungen und Präparate können mit anderen chemotherapeutischen oder chemopräventiven Mitteln kombiniert oder in Verbindung mit diesen verwendet werden. Die Zusammensetzungen und Präparate können beispielsweise gemeinsam mit herkömmlichen, im Stand der Technik bekannten Chemotherapeutika verabreicht werden, beispielsweise zwischen zwei aufeinanderfolgenden Zyklen einer Chemotherapie. Geeignete chemotherapeutische Mittel, die im Rahmen der vorliegenden Erfindung gemeinsam mit den Zusammensetzungen und Präparaten angewendet werden können, umfassen die gegenwärtig im Stand der Technik verfügbaren Mittel, einschließlich alkylierende Mittel; Alkylsulfonate; Aziridine, wie z.B. Thiotepa; Ethylenimine; Anti-Metaboliten; Folsäure-Analoga, wie z.B. Methotrexat (Farmitrexat®, Lantarel®, METEX®, MTX Hexal®); Purin-Analoga, wie z.B. Azathioprin (Azaiprin®, AZAMEDAC®, Imurek®, Zytrim®), Cladribin (Leustatin®), Fludarabinphosphat (Fludara®), Mercaptopurin (MER-CAP®, Puri-Nethol®), Pentostatin (Nipent®), Thioguanin (Thioguanin-Wellcome®) oder Fludarabin; Pyrimidin-Analoga, wie z.B. Cytarabin (Alexan®, ARA-cell®, Udicil®), Fluorouracil, 5-FU (Efudix®, Fluoroblastin®, Ribofluor®), Gemcitabin (Gemzar®), Doxifluridin, Azacitidin, Carmofur, 6-Azauridin, Floxuridin; Stickstoff-Lost-Derivate, wie z.B. Chlorambucil (Leukeran®), Melphalan (Alkeran®), Chlornaphazin, Estramustin, Mechlorethamin; Oxazaphosphorine, wie z.B. Cyclophosphamid (CYCLO-cell®, Cyclostin®, Endoxan®), Ifosfamid (Holoxan®, IFO-cell®) oder Trofosfamid (Ixoten®); Nitrosoharnstoffe, wie z.B. Bendamustin (Ribomustin ®), Carmustin (Carmubris®), Fotemustin (Muphoran®), Lomustin (Cecenu®, Lomeblastin®), Carmustin, Chlorozotocin, Ranimustin oder Nimustin (ACNU®); Hydroxyharnstoffe (Litalir®); Vincaalkaloide und Taxene, wie z.B. Vinblastin (Velbe®), Vindesin (Eldisine®), Vinorelbin (Navelbine®), Docetaxel (Taxotere®), oder Paclitaxel (Taxol®); Platin-Verbindungen, wie z.B. Cisplatin (Platiblastin®, Platinex®) oder Carboplatin (Carboplat®, Ribocarbo®); Sulfonsäureester, wie z.B. Busulfan (Myleran®), Piposulfan oder Treosulfan (Ovastat®); Anthrazykline, wie z.B. Doxorubicin (Adriblastin®, DOXO-cell®), Daunorubicin (Daunoblastin®), Epirubicin (Farmorubicin®), Idarubicin (Zavedos®), Amsacrin (Amsidyl®) oder Mitoxantron (Novantron®); sowie Derivate, Tautomere und pharmazeutisch aktive Salze der oben genannten Verbindungen. Auch eine Kombination der erfindungsgemäßen pharmazeutischen Zusammensetzungen oder Präparate im Rahmen von antiangiogenen Behandlungsansätzen, z.B. mit dem monoklonalen Antikörper Bevacizumab (Avastin®) oder mit Tyrosinkinase-Inhibitoren, wie z.B. Sorafenib (Nexavar®) oder Sunitinib (Sutent®), ist ohne weiteres möglich. Kombinationen der Zusammensetzungen oder Präparate mit therapeutischen Antikörpern, wie z.B. Trastuzumab (Herceptin®), Gemtuzumab (Mylotarg®), Panitumumab (Vectibix®) oder Edrecolomab (Panorex®), sind ebenfalls erfindungsgemäß umfasst.

Die erfindungsgemäßen pharmazeutischen Zusammensetzungen oder Präparate können darüber hinaus mit einer herkömmlichen Bestrahlungstherapie kombiniert werden. Die Bestrahlung kann dabei vor oder nach Verabreichung des erfindungsgemäßen Kombinationspräparats nach im Stand der Technik bekannten Verfahren durchgeführt werden. Die Bestrahlung kann Gammastrahlen, Röntgenstrahlen sowie die von Radioisotopen ausgesandte Strahlung umfassen. Die Bestrahlungstherapie kann des weiteren Teilchenstrahlung (Elektronen, Protonen, Neutronen) umfassen. Entsprechende Dosismengen, die bei der Behandlung von Tumoren eingesetzt werden, sind dem auf dem Gebiet der Strahlentherapie tätigen Fachmann ohne weiteres bekannt. Je nach Art des Tumors können Gesamtdosen von 20 bis 80 Gray zum Einsatz kommen.

Die erfindungsgemäßen Zusammensetzungen oder Präparate können (getrennt voneinander oder gemeinsam in einer einheitlichen Darreichungsform) in jeder im Stand der Technik bekannten, geeigneten Darreichungsform verabreicht werden. Solche Formulierungen richten sich im Wesentlichen nach den Eigenschaften des jeweils verwendeten Phosphodiesterasehemmers bzw. COX-Inhibitors und umfassen beispielsweise Formulierungen für eine orale, rektale, nasale oder parenterale (einschließlich subkutaner, intramuskulärer, intravenöser und intradermaler) Verabreichung. Gewöhnlich umfassen die erfindungsgemäßen pharmazeutischen Zusammensetzungen oder Präparate neben Phosphodiesterasehemmer(n) und COX-Inhibitor(en) einen oder mehrere pharmazeutisch akzeptable Träger, die mit den übrigen Inhaltsstoffen der Zusammensetzungen physiologisch kompatibel sind. Die erfindungsgemäßen Zusammensetzungen oder Präparate können neben den eigentlichen Wirkstoffen auch weitere Hilfsstoffe, Bindemittel, Verdünnungsmittel oder ähnliche Substanzen umfassen.

Die erfindungsgemäßen Zusammensetzungen und Präparate können mittels im Stand der Technik hinreichend bekannter Verfahren hergestellt werden. Derartige Verfahren sowie geeignete Hilfsstoffe und Träger werden beispielsweise in "Remington: The Science and Practice of Pharmacy", Lippincott Williams & Wilkins; 21. Auflage (2005) beschrieben. Die erfindungsgemäßen Zusammensetzungen sowie die Komponenten der erfindungsgemäßen Kombinationspräparate können beispielsweise in Form von Granulaten, Pulvern, Tabletten, Kapseln, Sirup, Suppositorien, Injektionen, Emulsionen, Suspensionen oder Lösungen vorliegen. Die Zusammensetzungen und die Komponenten der Präparate können für verschiedene Verabreichungsarten formuliert sein, beispielsweise für die orale, bukkale, sublinguale, transmukosale, rektale, subkutane, intrathekale, intravenöse, intramuskuläre, intraperitoneale, nasale, intraokulare oder intraventrikuläre Verabreichung. Die erfindungsgemäßen Zusammensetzungen und die Komponenten der Präparate können darüber hinaus auch als Retardmittel formuliert werden.

Für die orale, bukkale und sublinguale Verabreichung werden in der Regel feste Formulierungen wie, z.B. Pulver, Suspensionen, Granulate, Tabletten, Pillen, Kapseln und Gelcaps verwendet. Diese können beispielsweise hergestellt werden, indem man die Wirkstoffe oder deren Salze mit mindestens einem Additiv oder mit mindestens einem Hilfsstoff vermischt. Derartige Hilfsstoffe und Träger werden beispielsweise in "Remington: The Science and Practice of Pharmacy", Lippincott Williams & Wilkins; 21. Auflage (2005) beschrieben. Als Additive oder Hilfsstoffe können beispielsweise mikrokristalline Cellulose, Methylcellulose, Hydroxypropylmethylcellulose, Casein, Albumin, Mannit, Dextran, Saccharose, Lactose, Sorbitol, Stärke, Agar, Alginate, Pectine, Kollagen, Glyceride oder Gelatine verwendet werden. Ferner können orale Dosisformen Antioxidantien (z.B. Ascorbinsäure, Tocopherol oder Cystein), Gleitmittel (z.B. Magnesiumstearat), Konservierungsmittel (z.B. Paraben oder Sorbinsäure), Sprengmittel, Bindemittel, Verdicker, Geschmacksverbesserer, Farbstoffe und ähnliche Substanzen umfassen.

Flüssige Formulierungen, die sich für die orale Verabreichung eignen, können beispielsweise als Emulsion, Sirup, Suspension oder Lösungen vorliegen. Diese Formulierungen können unter Verwendung einer sterilen Flüssigkeit als pharmazeutischer Träger (z.B. Öl, Wasser, Alkohol oder Kombinationen derselben) in Form von flüssigen Suspensionen oder Lösungen hergestellt werden. Für die orale oder parenterale Verabreichung können pharmazeutisch geeignete Tenside, Suspensionsmittel, Öle oder Emulgatoren zugefügt werden. Für den Gebrauch in flüssigen Dosisformen geeignete Öle umfassen beispielsweise Olivenöl, Sesamöl, Erdnußöl, Rapsöl und Maisöl. Geeignete Alkohole umfassen Ethanol, Isopropylalkohol, Hexadecylalkohol, Glycerin und Propylenglykol. Suspensionen können ferner Fettsäureester, wie Ethyloleat, Isopropylmyristat, Fettsäureglyceride und acetylierte Fettsäureglyceride umfassen. Ferner werden Suspensionen häufig auch mit Substanzen wie Mineralöl oder Petrolatum versetzt.

Für die Injektion geeignete Formulierungen umfassen im Allgemeinen wässrige Suspensionen oder Ölsuspensionen, die unter Verwendung eines geeigneten Dispersionsmittels und eines Suspensionsmittels hergestellt werden können. Injizierbare Formen können in Lösungsphase oder in Form einer Suspension vorliegen, die mit einem Lösungsmittel oder Verdünnungsmittel hergestellt wird. Als pharmazeutischer Träger kommen u.a. sterilisiertes Wasser, Ringer-Lösung oder eine isotonische wäßrige Kochsalzlösung in Betracht. Alternativ dazu kann man als Träger sterile Öle einsetzen, die vorzugsweise nicht flüchtig sind. Für die Injektion geeignete Dosisformen umfassen ferner Pulver, die für die Rekonstitution mit einer geeigneten Lösung geeignet ist. Beispiele hierfür sind u.a. gefriergetrocknete oder sprühgetrocknete Pulver. Für die Injektion können diese Formulierungen gegebenenfalls mit Stabilisierungsmitteln oder Tensiden versetzt werden. Die Formulierungen können durch Injektion, wie durch Bolus-Injektion oder durch kontinuierliche Infusion verabreicht werden.

Für die rektale Verabreichung können die pharmazeutischen Zusammensetzungen oder die Komponenten der Präparate beispielsweise in Form eines Suppositoriums oder einer Salbe vorliegen. Suppositorien können durch Mischen der entsprechenden Wirkstoffe mit geeigneten Trägern hergestellt werden. Geeignete pharmazeutische Träger umfassen beispielsweise Kakaobutter, Glyceride oder Polyethylenglykol. Diese Substanzen liegen bei normalen Lagerungstemperaturen in fester Form vor, wohingegen sie bei der Freisetzung im Rektum in die flüssige Form übergehen. Bei der Herstellung von Suppositorien können auch Öle eingesetzt werden.

Die am besten für die jeweilige Therapie geeigneten Verabreichungsarten und die zu verabreichenden Mengen von COX-Inhibitor und Phosphodiesterasehemmer können vom Fachmann problemlos mittels Routineverfahren ausfindig gemacht werden. Faktoren, die-die jeweilige Menge von COX-Inhibitor und Phosphodiesterasehemmer in der zu verabreichenden pharmazeutischen Zusammensetzung beeinflußen, umfassen die Art und Schwere der Krebserkrankung, das Alter, Körpergewicht, Geschlecht und den allgemeinen Gesundheitszustand des zu behandelnden Patienten, die gleichzeitige Verabreichung anderer therapeutischer Mittel und andere Faktoren.

Geeignete pharmazeutische Zusammensetzungen und Präparate zur humanen Verabreichung werden in der Regel 0,01 mg bis 10 mg COX-Inhibitor pro kg Körpergewicht des Patienten umfassen. Vorzugsweise liegt die Menge des COX-Inhibitors im Bereich von 0,1 mg bis 5 mg pro kg Körpergewicht des Patienten und noch stärker bevorzugt im Bereich von 0,5 mg bis 1 mg pro kg Körpergewicht des Patienten. Die Menge an Phosphodiesterasehemmer in den pharmazeutischen Zusammensetzungen oder Präparaten wird üblicherweise von 0,01 mg bis 50 mg pro kg Körpergewicht des Patienten betragen. Vorzugsweise wird die Menge an Phosphodiesterasehemmer in den erfindungsgemäßen Zusammensetzungen oder Präparaten im Bereich von 0,05 mg bis 25 mg pro kg Körpergewicht des Patienten und noch stärker bevorzugt im Bereich von 0,1 mg bis 10 mg pro kg Körpergewicht des Patienten liegen.

Die therapeutische Wirksamkeit der erfindungsgemäßen pharmazeutischen Zusammensetzungen und Präparate kann anhand von im Stand der Technik bekannten Parametern bewertet werden. Diese Parameter umfassen u.a. die Wirksamkeit der erfindungsgemäßen Zusammensetzung bei der Beseitigung von Tumoren, die Ansprechrate, die Zeit bis zum Fortschreiten der Erkrankung und die Überlebensrate der behandelten Patienten. Eine gegen einen Tumor gerichtete Wirkung kann sich durch eine Inhibierung des Tumorwachstums, eine Verzögerung des Tumorwachstums, eine Verkleinerung des Tumors, eine Verringerung der Anzahl der Tumorzellen, eine Verlängerung der Zeitspanne bis zum Einsetzen eines erneuten Tumorwachstums sowie eine Verzögerung im Fortschreiten der Erkrankung darstellen.

Vorzugsweise führen die erfindungsgemäßen Zusammensetzungen und Präparate zu einem vollständigen Ansprechen beim Patienten. Mit vollständigem Ansprechen ist die Beseitigung sämtlicher klinisch nachweisbarer Erkrankungssymptome sowie die Wiederherstellung von normalen Befunden im Blutbild, bei Röntgenuntersuchungen, CT-Aufnahmen und Ähnlichem gemeint. Ein solches Ansprechen dauert mindestens einen Monat nach Absetzen der erfindungsgemäßen Behandlung an. Die antiproliferativ wirksamen Zusammensetzungen und Präparate der vorliegenden Erfindung können alternativ dazu auch zu einem partiellen Ansprechen beim Patienten führen. Bei einem partiellen Ansprechen kommt es zu einer Verringerung der meßbaren Tumorbelastung im Patienten. Vorzugsweise nimmt die Anzahl der im Patienten vorhandenen Tumorzellen ab, und es werden keine neuen Läsionen beobachtet. Gleichzeitig kommt es vorzugsweise zu einer Verbesserung eines oder mehrerer durch die Krankheit verursachter Symptome (z.B. Fieber, Gewichtsverlust, Erbrechen).

Die Kombination aus Phosphodiesterasehemmer und COX-Inhibitor führt zu einer synergistischen, antiproliferativen Wirkung, die einen Einsatz der erfindungsgemäßen Kombination als cytostatisches Mittel bei der Behandlung von Krebs ermöglicht. Wie vorliegend verwendet ist unter einer synergistischen Wirkung eine Wirkung zu verstehen, die stärker ist als durch einfache Addition der bei alleiniger Gabe der Komponenten beobachteten Effekte zu erwarten gewesen wäre. Eine synergistische Wirkung erlaubt die Verabreichung von geringeren Mengen von Phosphodiesterasehemmer bzw. COX-Inhibitor, sodass die Verträglichkeit des erfindungsgemäßen Kombinationspräparates für den Patienten in der Regel problemlos ist. So können die einzelnen Komponenten der Kombination, d.h. der oder die Phosphodiesterasehemmer und der oder die COX-Inhibitor(en), ggf. in subtherapeutischen Dosen verabreicht werden, die bei alleiniger Gabe keine Wirkung auf den Verlauf der zu behandelnden Krebserkrankung zeigen würden.

In einem weiteren Aspekt betrifft die Erfindung die Verwendung eines wie oben definierten Phosphodiesterasehemmers zur Herstellung einer pharmazeutischen Zusammensetzung, die mindestens einen Phosphodiesterasehemmer und mindestens einen COX-Inhibitor umfasst, für die Behandlung einer Krebserkrankung. Die Erfindung betrifft somit auch die Verwendung eines COX-Inhibitors zur Herstellung einer pharmazeutischen Zusammensetzung, die mindestens einen COX-Inhibitor und mindestens einen Phosphodiesterasehemmer umfasst, für die Behandlung einer wie oben definierten Krebserkrankung. Entsprechend betrifft die Erfindung auch die Verwendung eines wie oben definierten Phosphodiesterasehemmers zur Herstellung eines pharmazeutischen Kombinationspräparats für die gleichzeitige oder zeitlich getrennte Anwendung in der Therapie, wobei das Präparat mindestens einen Phosphodiesterasehemmer und mindestens einen COX-Inhibitor umfasst. Die Erfindung betrifft auch die Verwendung eines wie oben definierten COX=Inhibitors zur Herstellung eines pharmazeutischen Kombinationspräparats für die gleichzeitige oder zeitlich getrennte Anwendung in der Therapie, wobei das Präparat mindestens einen Phosphodiesterasehemmer und mindestens einen COX-Inhibitor umfasst.

Bei dem zu verwendenden Phosphodiesterasehemmer handelt es sich wie oben beschrieben vorzugsweise um einen Phosphodiesterasehemmer aus der Gruppe bestehend aus 1-Methyl-3-isobutylxanthin (IBMX), Theophyllin, CC-8062, CC-8075, Vinpocetin, Anagrelid, EHNA (Erythro-9-(2-hydroxy-3-nonyl)adenin), Milrinon, Enoximon, Mesembrin, Rolipram, Ibudilast, Piclamilast, Pentoxyfyllin, Sildenafil, Tadalafil, Vardenafil, Udenafil, Avanafil, Dipyridamol sowie pharmazeutisch geeigneten Derivaten, Salzen oder Solvaten derselben. Besonders bevorzugt wird die Verwendung der Phosphodiesterasehemmer 1-Methyl-3-isobutylxanthin und/oder Sildenafil oder pharmazeutisch geeigneten Derivaten, Salzen oder Solvaten derselben. Bei dem zu verwendenden COX-Inhibitor handelt es sich wie oben beschrieben vorzugsweise um einen solchen, der ausgewählt ist aus der Gruppe bestehend aus Indometacin, Meloxicam, Valdecoxib, Celecoxib, Rofecoxib, Aspirin, Ibuprofen, NS-398, Nimesulid sowie pharmazeutisch geeigneten Derivaten, Salzen oder Solvaten derselben. Besonders bevorzugt wird die Verwendung der COX-Inhibitoren Indometacin und/oder Meloxicam oder pharmazeutisch geeigneten Derivaten, Salzen oder Solvaten derselben. Bei der Krebserkrankung, die mit Hilfe des pharmazeutischen Kombinationspräparats behandelt werden soll, kann es sich um eine beliebige der oben aufgeführten Krebserkrankung handeln. Besonders bevorzugt ist es, dass es sich bei der zu behandelnden Krebserkrankung um ein Bronchialkarzinom handelt, insbesondere um ein kleinzelliges Bronchialkarzinom.

Die Erfindung betrifft in einem weiteren Aspekt ein Verfahren zur Behandlung eines Patienten, der an einer Krebserkrankung leidet, bei dem man dem Patienten mindestens einen Phosphodiesterasehemmer und mindestens einen COX-Inhibitor verabreicht. Wie oben beschrieben handelt es sich bei dem Phosphodiesterasehemmer vorzugsweise um 1-Methyl-3-isobutylxanthin und/oder Sildenafil oder um pharmazeutisch geeignete Derivate, Salze oder Solvate derselben. Bei dem COX-Inhibitor handelt es sich vorzugsweise um Indometacin und/oder Meloxicam oder um davon abgeleitete pharmazeutisch geeignete Derivate, Salze oder Solvate. Die Verabreichung des Phosphodiesterasehemmers kann gleichzeitig oder zeitlich getrennt von der Verabreichung des COX-Inhibitors erfolgen. Dies bedeutet, dass die Kombination aus Phosphodiesterasehemmers und COX-Inhibitor wie oben beschrieben entweder in einer einheitlichen Darreichungsform (d.h. in einer einzigen pharmazeutischen Zusammensetzung) vorliegen kann, die an den Patienten verabreicht wird, oder in getrennten Darreichungsformen, die zu verschiedenen Zeitpunkten, d.h. zeitlich getrennt voneinander, an den Patienten verabreicht werden.

In einer bevorzugten Ausführungsform der vorliegenden Erfindung wird der Phosphodiesterasehemmer vor Verabreichung des COX-Inhibitors an den Patienten verabreicht. Vorzugsweise wird die Verabreichung des Phosphodiesterasehemmers 1-24 h vor Verabreichung des COX-Inhibitors durchgeführt. In einer besonders bevorzugten Ausführungsform wird die Verabreichung des Phosphodiesterasehemmers 12-16 h vor Verabreichung des COX-Inhibitors durchgeführt. In einer noch weiteren Ausführungsform der vorliegenden Erfindung wird der COX-Inhibitor vor Verabreichung des Phosphodiesterasehemmers an den Patienten verabreicht. Vorzugsweise wird die Verabreichung des COX-Inhibitors 1-24 h vor Verabreichung des Phosphodiesterasehemmers durchgeführt. In einer besonders bevorzugten Ausführungsform wird die Verabreichung des COX-Inhibitors 12-16 h vor Verabreichung des oder der Phosphodiesterasehemmer durchgeführt.

Schließlich betrifft die Erfindung einen Kit, der die folgenden Bestandteile umfasst:
a) mindestens einen Phosphodiesterasehemmer,
b) mindestens einen COX-Inhibitor, und (optional)
c) Anweisungen zur kombinierten Verwendung des Phosphodiesterasehemmers und des COX-Inhibitors bei der Behandlung eines Patienten mit einer Krebserkrankung.

Der Phosphodiesterasehemmer des Kits ist vorzugsweise ausgewählt aus der Gruppe bestehend aus 1-Methyl-3-isobutylxanthin (IBMX), Theophyllin, CC-8062, CC-8075, Vinpocetin, Anagrelid, EHNA (Erythro-9-(2-hydroxy-3-nonyl)adenin), Milrinon, Enoximon, Mesembrin, Rolipram, Ibudilast, Piclamilast, Pentoxyfyllin, Sildenafil, Tadalafil, Vardenafil, Udenafil, Avanafil, Dipyridamol und pharmazeutisch geeigneten Salzen oder Solvaten davon. Besonders bevorzugt handelt es sich bei dem Phosphodiesterasehemmer um 1-Methyl-3-isobutylxanthin und/oder Sildenafil oder um ein pharmazeutisch geeignetes Derivat, Salz oder Solvat davon. Der im Kit enthaltende COX-Inhibitor ist ausgewählt aus der Gruppe bestehend aus Indometacin, Meloxicam, Valdecoxib, Celecoxib, Rofecoxib, Aspirin, Ibuprofen, NS-398, Nimesulid und pharmazeutisch geeigneten Salzen oder Solvaten davon. Besonders bevorzugt handelt es sich bei dem COX-Inhibitor um Indometacin und/oder Meloxicam oder ein pharmazeutisch geeignetes Derivat, Salz oder Solvat dieser Verbindungen.

Die Anweisungen zur kombinierten Verwendung des Phosphodiesterasehemmers und COX-Inhibitors bei der Behandlung eines Patienten mit einer Krebserkrankung beschreiben dem Anwender die Schritte, die bei der Herstellung und Verabreichung der erfindungsgemäßen pharmazeutischen Zusammensetzungen und Präparate durchzuführen sind, um zu dem gewünschten therapeutischen Ergebnis zu gelangen. Die Anweisungen können beispielsweise spezifische Mengen und Behandlungspläne umfassen, und sie können Informationen bezüglich der Verabreichungsdauer einschließen. Der Kit kann darüber hinaus andere Bestandteile umfassen, die zur Durchführung der vorliegenden Erfindung nützlich sind. Der Kit kann beispielsweise geeignete Lösungsmittel, Hilfsstoffe; Bindemittel, Verdünnungsmittel oder ähnliche Substanzen umfassen. Ferner kann der Kit auch Injektionsspritzen, Kanülen, Katheter und andere Hilfsmittel, die für die Verabreichung der erfindungsgemäßen pharmazeutischen Zusammensetzungen und Präparate nützlich sind, umfassen.

Die pharmakologisch wirksamen Substanzen können im Kit in jeder beliebigen Form bereitgestellt werden, die ihre pharmakologische Aktivität erhält. So lassen sich Phosphodiesterasehemmer und COX-Inhibitor beispielsweise in gelöster oder lyophilisierter Form bereitstellen.

Wenn der/die Phosphodiesterasehemmer und der/die COX-Inhibitor(en) als getrennte Bestandteile innerhalb eines Kits bereitgestellt werden, so liegen diese vorzugsweise in getrennten Behältern vor, z.B. Fläschchen oder Ampullen. Die Inhalte der Behälter können bei Bedarf miteinander vermischt werden, z.B. durch Entfernen des Inhalts eines Behälters und Überführung desselben in den anderen Behälter. Alternativ dazu können beide Behälter zur Vermischung von Phosphodiesterasehemmer und COX-Inhibitor in einen dritten Behälter überführt werden. So kann z.B. ein Wirkstoff-Bestandteil des Kits, z.B. ein Phosphodiesterasehemmer oder ein COX-Inhibitor, in einer Injektionsspritze vorliegen, während der andere Wirkstoff-Bestandteil in einem Behälter, wie z.B. in einem Fläschchen oder in einer Ampulle, vorliegt. Beim Aufziehen des Inhalts des Behälters (ggf. nach Zugabe eines entsprechenden Lösungsmittels) kommt es zur Mischung der beiden Wirkstoff-Bestandteile in der Spritze. Die Wirkstoff-Bestandteile der erfindungsgemäßen Kombination können ferner in einer Spritze mit zwei getrennten Kammern enthalten sein, sodass diese bis zur Verabreichung getrennt voneinander vorliegen. Bei der Betätigung der Spritze im Rahmen der Verabreichung werden die Inhalte der beiden Kammern miteinander vermischt. Die Wirkstoff-Bestandteile der erfindungsgemäßen Kits liegen vorzugsweise in flüssiger, emulgierter oder lyophilisierter Form in den Behältern oder Spritzen vor. Lyophilisierte Wirkstoff-Bestandteile werden in der Regel üblicherweise weitere Stabilisationsmittel, wie beispielsweise Laktose oder Mannitol enthalten.

### BESCHREIBUNG DER FIGUREN

Figur 1 zeigt die Eichkurven der Zelllinien OH1, OH3, H69, SW2 und H82. Aufgeführt ist die Extinktion bei sieben verschiedenen Zellzahlen zwischen 10⁴ und 10⁷ Zellen pro ml.
Figur 2 zeigt die Behandlung von OH1-Zellen mit Indometacin (400-800 µM) und IBMX (0,125-2,0 mM). Gezeigt ist der Mittelwert ± SEM, n ≥ 6, One-Way-ANOVA (Post-Test: Bonferroni's Multiple Comparison Test) * p < 0,05; ** p < 0,005; *** p < 0,001.
Figur 3 zeigt die Behandlung von OH3-Zellen mit Indometacin (50-300 µM) und IBMX (0,125-2,0 mM). Gezeigt ist der Mittelwert ± SEM, n ≥ 10, One-Way-ANOVA (Post-Test: Bonferroni's Multiple Comparison Test) * p < 0,05; ** p < 0,005; *** p < 0,001.
Figur 4 zeigt die Behandlung von OH3-Zellen mit Indometacin (400-800 µM) und IBMX (0,125-2,0 mM). Gezeigt ist der Mittelwert ± SEM, n ≥ 6, One-Way-ANOVA (Post-Test: Bonferroni's Multiple Comparison Test) * p < 0,05; ** p < 0,005; *** p < 0,001.
Figur 5 zeigt die Behandlung von H69-Zellen mit Indometacin (50-300 µM) und IBMX (0,125-2,0 mM). Gezeigt ist der Mittelwert ± SEM, n ≥ 9, One-Way-ANOVA (Post-Test: Bonferroni's Multiple Comparison Test) * p < 0,05; ** p < 0,005; *** p < 0,001.
Figur 6 zeigt die Behandlung von H69-Zellen mit Indometacin (400-800 µM) und IBMX (0,125-2,0 mM). Gezeigt ist der Mittelwert ± SEM, n ≥ 6, One-Way-ANOVA (Post-Test: Bonferroni's Multiple Comparison Test) * p < 0,05; ** p < 0,005; *** p < 0,001.
Figur 7 zeigt die Behandlung von SW2-Zellen mit Indometacin (50-300 µM) und IBMX (0,125-2,0 mM). Gezeigt ist der Mittelwert ± SEM, n ≥ 9, One-Way-ANOVA (Post-Test: Bonferroni's Multiple Comparison Test) * p < 0,05; ** p < 0,005; *** p < 0,001.
Figur 8 zeigt die Behandlung von SW2-Zellen mit Indometacin (400-800 µM) und IBMX (0,125-2,0 mM). Gezeigt ist der Mittelwert ± SEM, n ≥ 6, One-Way-ANOVA (Post-Test: Bonferroni's Multiple Comparison Test) * p < 0,05; ** p < 0,005; *** p < 0,001.
Figur 9 zeigt die Behandlung von H82-Zellen mit Indometacin (100-200 µM) und IBMX (0,015625-0,5 mM). Gezeigt ist der Mittelwert ± SEM, n = 12, One-Way-ANOVA (Post-Test: Bonferroni's Multiple Comparison Test) * p < 0,05; ** p < 0,005; *** p < 0,001.
Figur 10 zeigt die Behandlung von H69 Zellen mit Meloxicam (50-200 µM) (a), mit Sildenafil (125-500 µM) (b) und mit einer Kombination aus beiden Wirkstoffen (c). Die synergistische Wirkung von beiden Substanzen wird besonders bei der Kombination von 250 µM Sildenafil mit 50 µM Meloxicam deutlich.

### BEISPIELE

### Beispiel 1: Zellkulturverfahren

Die Wirkung der erfindungsgemäßen pharmazeutischen Zusammensetzungen auf das Wachstum von Tumorzellen wurde an den Zelllinien des kleinzelligen Bronchialkarzinoms H69, SW2, OH1, OH3 und H82 untersucht.

Die Zellen wurden *in vitro* unter Standardbedingungen (37°C, 100% relative Luftfeuchtigkeit, 5% CO₂) in Kulturmedium kultiviert. Ein Wechsel des Kulturmediums erfolgte einmal die Woche. Dafür wurde zunächst der Boden der Zellkulturflasche gründlich abgespült, so dass sich möglichst alle Zellen in Suspension mit dem Kulturmedium befanden. Dann wurden aus der Zellkulturflasche ca. 10 ml Zellsuspension abpipettiert und in ein 30 ml Röhrchen gegeben. Dieses wurde dann für 10 Minuten bei 1500 UpM (Umdrehungen/Minute) zentrifugiert. Anschließend wurde der Überstand abgesaugt und das Zellpellet mit frischem Kulturmedium resuspendiert. Diese Zellsuspension wurde in eine neue Zellkulturflasche gegeben, in die bereits ca. 15 ml Kulturmedium vorgelegt worden waren. Je nach Wachstum der Zellen wurde an zwei weiteren Tagen der Woche eine gewisse Menge der Zellsuspension aus der Zellkulturflasche abgesaugt und frisches Kulturmedium in die Flasche nachgefüllt.

### Beispiel 2: Bestimmung der Zellzahl

Die Anzahl der vitalen Zellen pro ml wurde mit Hilfe eines Zellzählgeräts (Casy®, Cell Counter und Analyzer System Modell TT, Schärfe System, Reutlingen, Deutschland) bestimmt. Dabei werden nach dem Prinzip der Pulsflächen-Analyse Veränderungen in der Membranintegrität detektiert, wodurch tote von vitalen Zellen unterschieden werden können.

Für die Bestimmung der Zellzahl wurden jeweils etwa 10 ml Zellsuspension in ein 30 ml Röhrchen pipettiert. Dieses wurde bei 1500 UpM für 10 Minuten zentrifugiert. Der Überstand wurde abgesaugt, und das Zellpellet wurde in frischem Kulturmedium resuspendiert. Diese Zellsuspension wurde durch ein Feinsieb ("cell-strainer cap") gegeben, um große aggregierte Zellen voneinander zu trennen. 100 µl der gesiebten Lösung wurden in ein mit 10 ml Zellkultur-Verdünnungslösung Casyton® (Schärfe System, Reutlingen, Deutschland) gefülltes kleines Plastikreagenzgefäß ("Casycup") pipettiert. Nach zweimaligem Invertieren des Casycups wurde mittels des Zellzählgeräts die Zahl der vitalen Zellen bestimmt.

Die statistisch signifikanten Unterschiede der Mittelwerte wurden durch einen ungepaarten t-Test nachgewiesen und als signifikant betrachtet, wenn p < 0,05 war. Dafür wurde vorausgesetzt, dass die Daten normalverteilt waren und gleiche Varianzen hatten.

### Beispiel 3: Erstellen von Eichkurven

Vor Beginn der Versuche zur Zellproliferation wurde zunächst für jede Zelllinie eine Eichkurve erstellt, um die optimale Zellzahl pro ml für die nachfolgenden Versuchsreihen zu ermitteln. Dafür wurde das Zellproliferationskit XTT (Roche, Penzberg, Deutschland) verwendet. Hierbei wird durch ein in allen vitalen Zellen vorhandenes Enzym das gelbe Tetrazoliumsalz XTT zum orangen Salz Formazan reduziert. Somit stellt die Extinktion der jeweiligen Zellkulturprobe ein Maß für die Anzahl der vitalen Zellen dar.

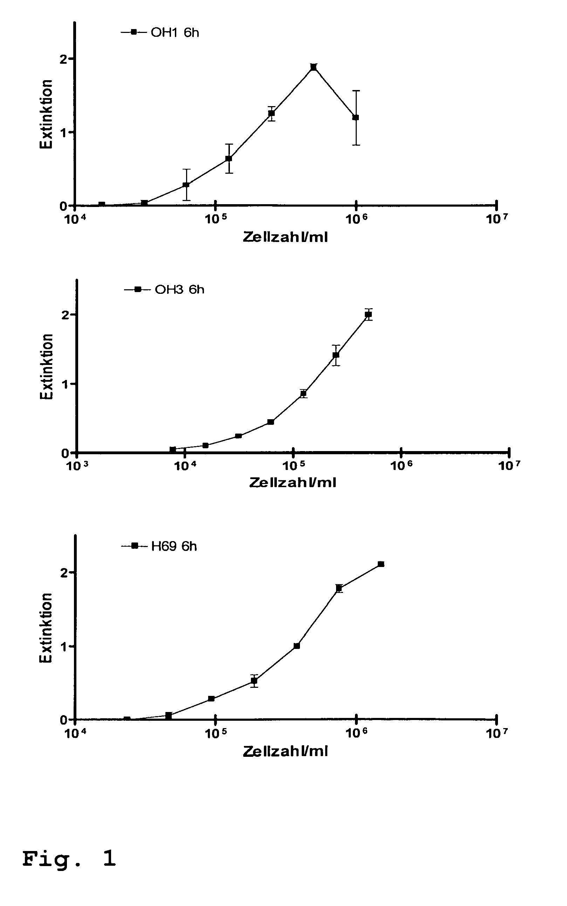

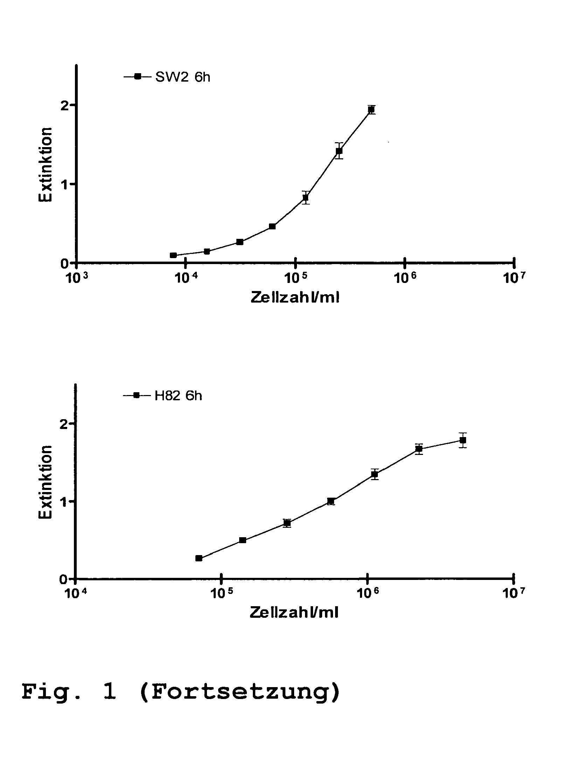

Die Zelllinien wurden in sieben verschiedenen Konzentrationen zwischen 10⁴ und 10⁷ Zellen pro ml in Mikrotiterplatten mit 96 Vertiefungen ausgesät. In jede Vertiefung wurde zunächst 10 µl PBS (pH 7,4) vorgelegt und dann 90 µl der Zellsuspension mit der jeweiligen Zellzahl pro ml zugegeben. Für jede Zelllinie wurden sechs parallele Ansätze hergestellt. Nach 72 Stunden Inkubation unter Standardbedingungen wurden 50 µl XTT Labeling Mixture pro Vertiefung zugegeben. Nach sechs Stunden wurde die Absorption bei 450 nm in einem Dynatech MR 3.13 Mikroelisa Reader gemessen und anhand der erzielten Werte Eichkurven erstellt. Figur 1 zeigt die Eichkurven für die Zelllinien H69, SW2, OH1, OH3 und H82OH1-Zellen. Die Zellzahlen wurden so gewählt, dass sich die Zellen bei ungehindertem Wachstum in der exponentiellen Wachstumsphase befanden. So ergab sich eine Konzentration von 160.000 Zellen pro ml für die Zelllinien OH3 und SW2, 300.000 Zellen pro ml für OH1, 500.000 Zellen pro ml für H69 und 750.000 Zellen pro ml für H82.

### Beispiel 4: In vitro Versuche mit Indometacin oder IBMX

Zunächst wurden Indometacin und IBMX einzeln zu den zu untersuchenden Zellen gegeben, um eine antiproliferative Wirkung nachzuweisen. Dieser Versuch wurde mit allen oben genannten Zelllinien durchgeführt. Dafür wurden zunächst Lösungen der einzelnen Wirkstoffe in die Vertiefungen einer 96-Loch-Mikrotiterplatte gegeben. Die in Kulturmedium vorliegenden Zellsuspensionen wurden zugegeben, sodass sich ein Gesamtvolumen von 100 µl pro Vertiefung ergab.

Die Zellzahl für die jeweiligen Zelllinien betrug für SW2 205.700 Zellen pro ml, für H82 964.000 Zellen pro ml, für OH1 385.700 Zellen pro ml, für OH3 205.700 Zellen pro ml und für H69 643.000 Zellen pro ml. Da zum Lösen von Indometacin 5% Ethanol und zum Lösen von IBMX 1 % Ethanol verwendet wurde, bestand die Kontrolle aus 70 µl Zellsuspension mit der jeweiligen Zellzahl pro ml, 20 µl Medium und 10 µl Alkoholkontrolle mit einer Konzentration von 6% Ethanol. Im Gesamtvolumen von 100 µl ergab sich somit eine Alkoholkonzentration von 0,6%. Der Leerwert bestand aus 100 µl Medium. Jede gewählte Kombination der Wirkstoffe in den unterschiedlichen Konzentrationen wurde pro Versuch sechsfach getestet.

Bei Gabe von geringen Konzentrationen Indometacin im Bereich von 50 µM bis 300 µM ließ sich feststellen, dass eine signifikante Inhibierung auf einen Wert von etwa 60% der Alkoholkontrolle sowohl bei 200 µM als auch bei 300 µM auftrat (Daten nicht gezeigt). Die Hemmung durch IBMX war ab einer Konzentration von 0,75 mM zu beobachten und betrug etwa 80 % der Alkoholkontrolle (Daten nicht gezeigt). Die durch IBMX verursachte Hemmung nahm bei Konzentrationen ab 1,25 mM zu und betrug 52 % der Alkoholkontrolle bei 1,25 mM IBMX, 51% der Alkoholkontrolle bei 1,5 mM IBMX und 45% der Alkoholkontrolle bei 2,0 mM IBMX. Beide Wirkstoffe zeigten folglich eine antiproliferative Wirkung auf die verschiedenen Zellen des kleinzelligen Bronchialkarzinoms.

### Beispiel 5: In vitro Versuche mit Kombinationen aus Indometacin und IBMX

Die zu untersuchenden Zelllinien wurden mit den in den Figuren angegeben Konzentrationen von Indometacin und IBMX behandelt. Die Proliferationsversuche wurden in 96-Loch-Mikrotiterplatten durchgeführt; das Gesamtvolumen pro Vertiefung betrug 100 µl. 80 µl Zellsuspension wurde pro Vertiefung verwendet. Die Zellzahl der jeweils verwendeten Suspensionen betrug für SW2 180.000 Zellen pro ml, für OH1 338.000 Zellen pro ml, für OH3 180.000 Zellen pro ml und für H69 563.000 Zellen pro ml. Da zum Lösen sowohl von Indometacin 20% Ethanol als auch von IBMX 20% Ethanol verwendet wurde, bestand die Kontrolle aus 80 µl Zellsuspension mit der jeweiligen Zellzahl pro ml und 20 µl Alkoholkontrolle mit einer Konzentration von 20% Ethanol. Im Gesamtvolumen von 100 µl ergab sich so eine Alkoholkonzentration von 4%. Eine weitere Kontrolle bestand aus 80 µl Zellsuspension mit der jeweiligen Zellzahl pro ml und 20 µl Kulturmedium, um den wachstumshemmenden Effekt vom Ethanol erkennen zu können. Der Leerwert bestand aus 100 µl Kulturmedium.

Die Zelllinie H82 wurde mit folgenden Konzentrationen behandelt: 200 µM, 100 µM, 50 µM, 25 µM, 12,5 µM und 6,25 µM Indometacin sowie 0,5 mM, 0,25 mM, 0,125 mM, 0,0625 mM, 0,03125 mM und 0,015625 mM IBMX. Die Kontrolle bestand in diesem Fall aus 80 µl Zellsuspension mit 844.000 Zellen pro ml und 20 µl Alkoholkontrolle mit einer Konzentration von 5% Ethanol. Im Gesamtvolumen von 100 µl ergab sich hier also eine Alkoholkonzentration von 1%. Die weitere Kontrolle ohne Alkohol und der Leerwert wurden hier genauso wie bei den oben beschriebenen Zelllinien durchgeführt.

Jede Kombination der beiden Wirkstoffe in den unterschiedlichen Konzentrationen wurde pro Versuch vierfach getestet (n = 3), sodass sich jeder Messpunkt aus 12 Einzelwerten zusammensetzte. Für diesen Versuch wurde ein One-Way-Anova und anschließend ein Bonferroni's Multiple Comparison Test durchgeführt. Alle graphisch dargestellten Daten sind als Mittelwert ± SEM (Standardfehler des Mittelwertes) aufgeführt.

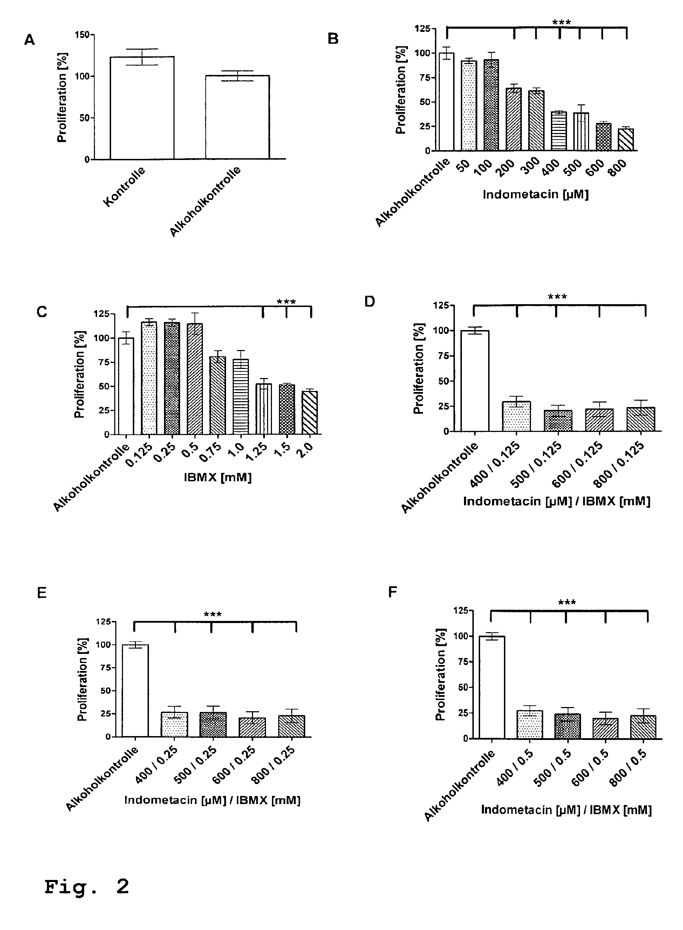

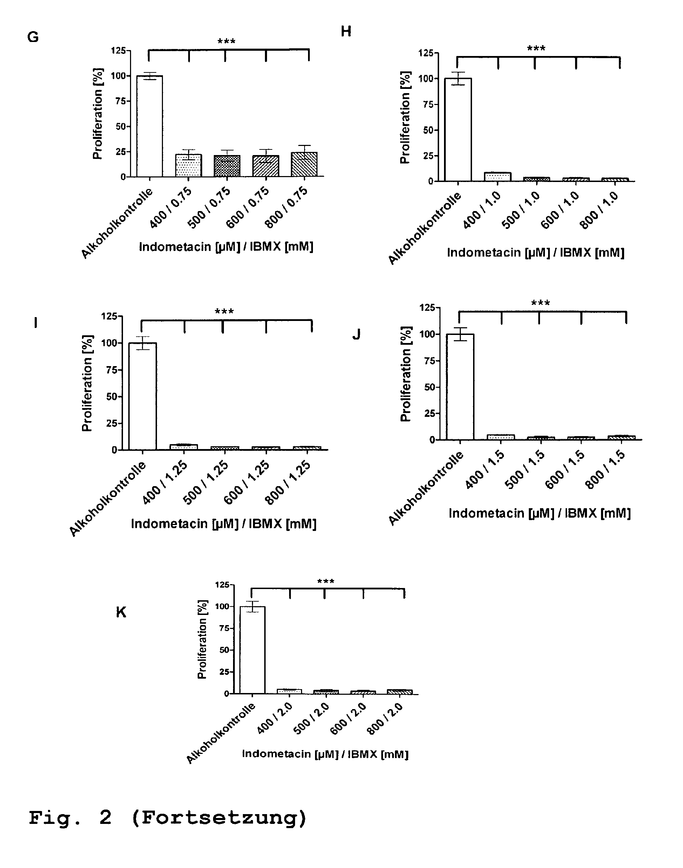

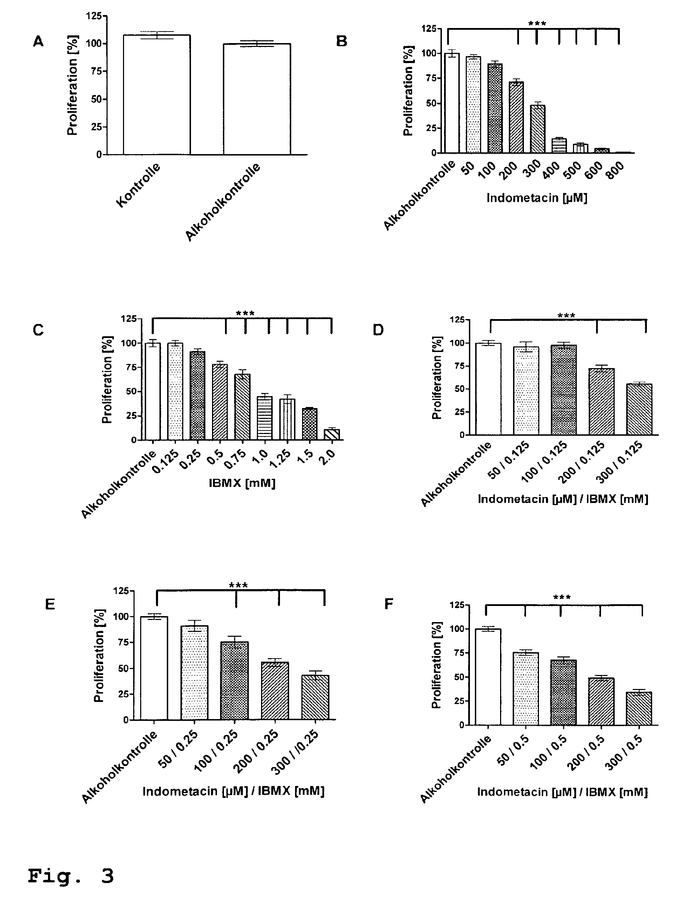

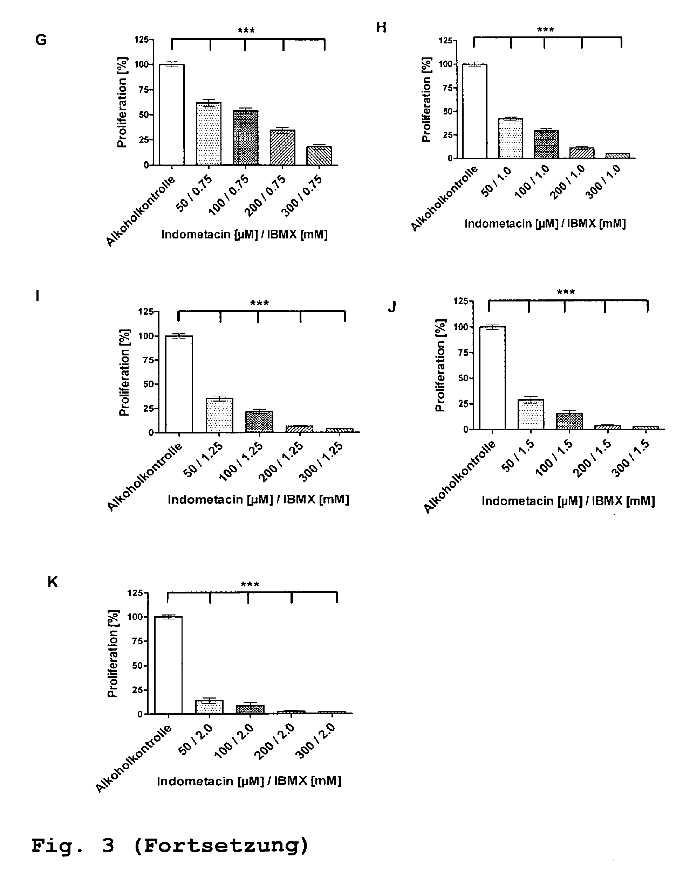

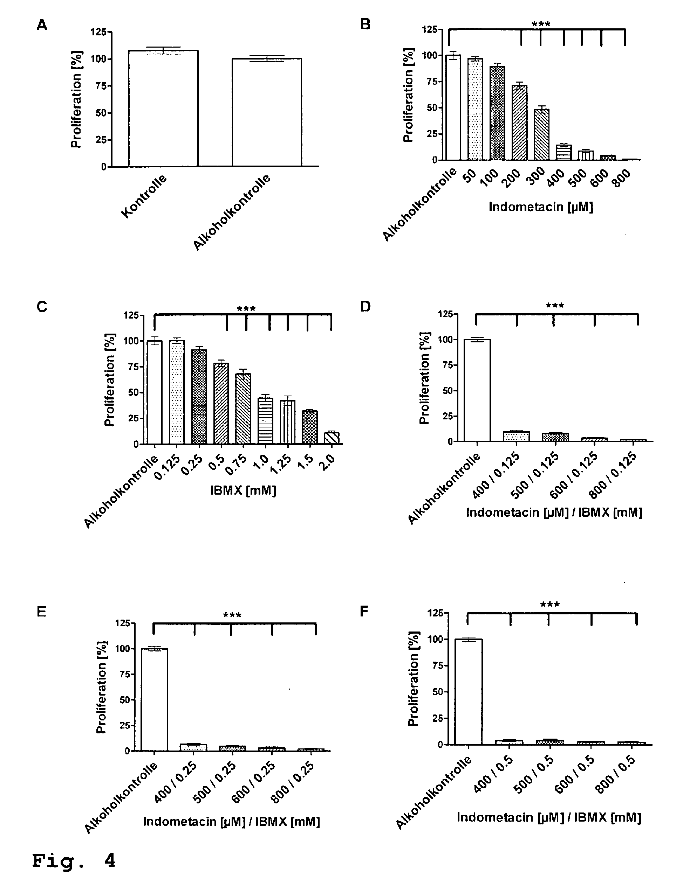

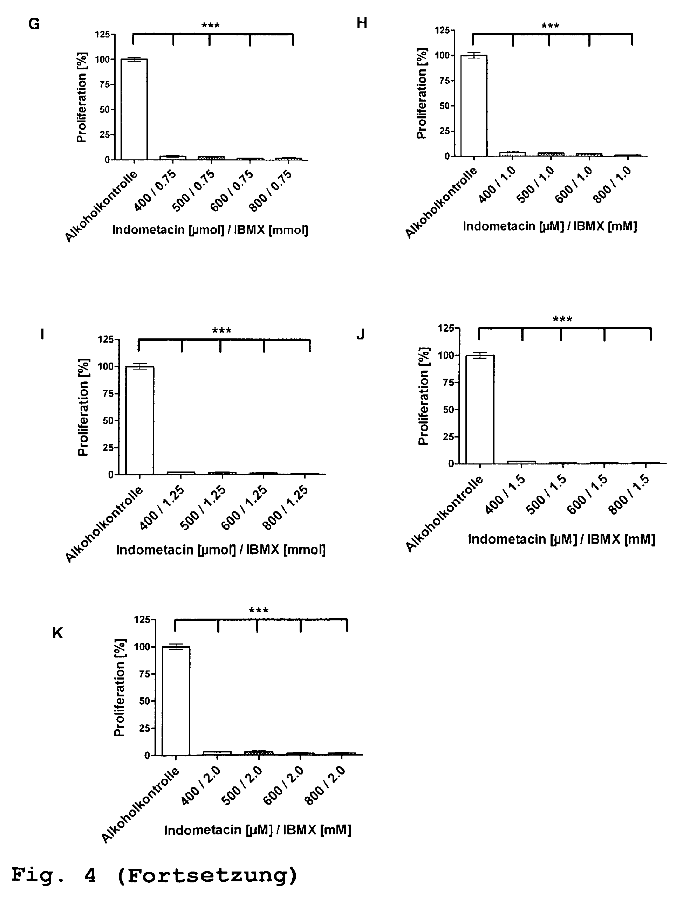

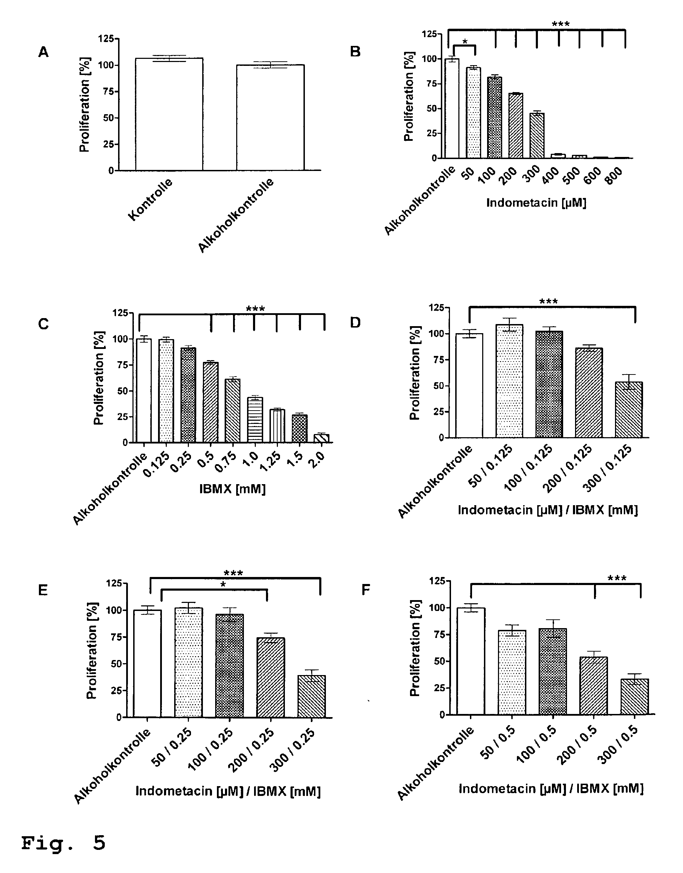

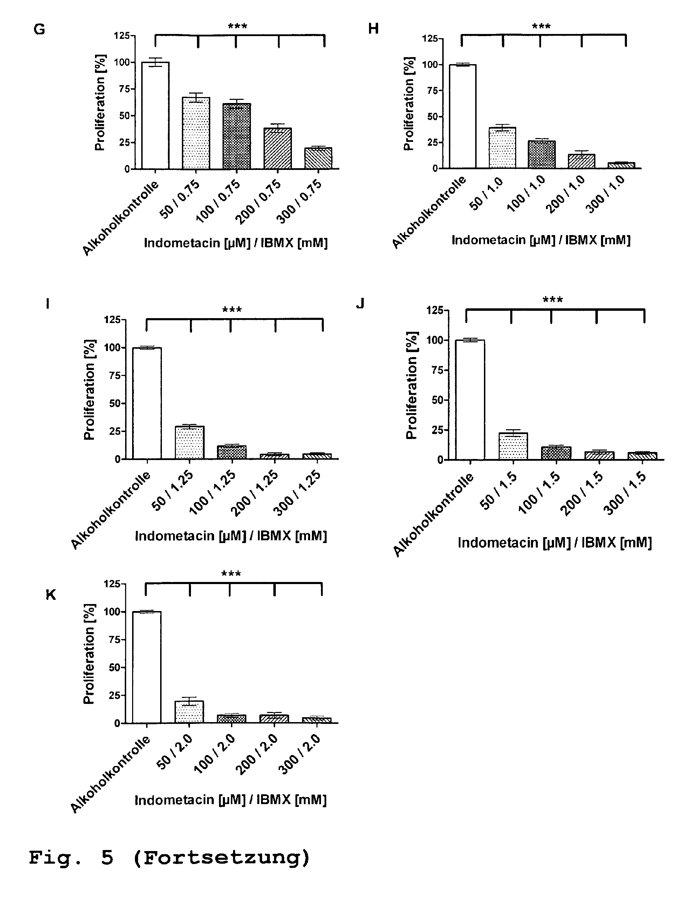

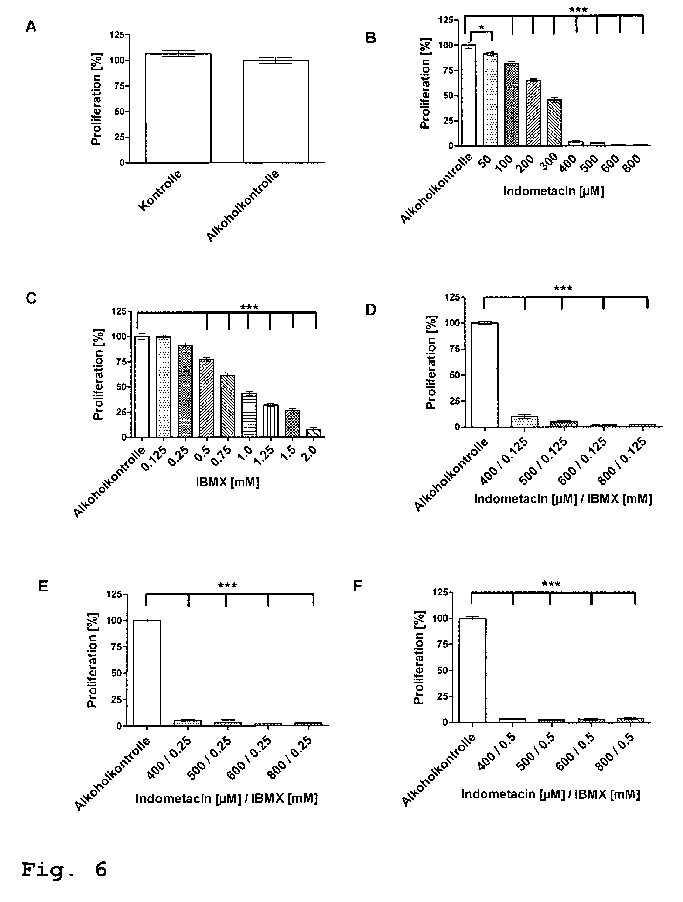

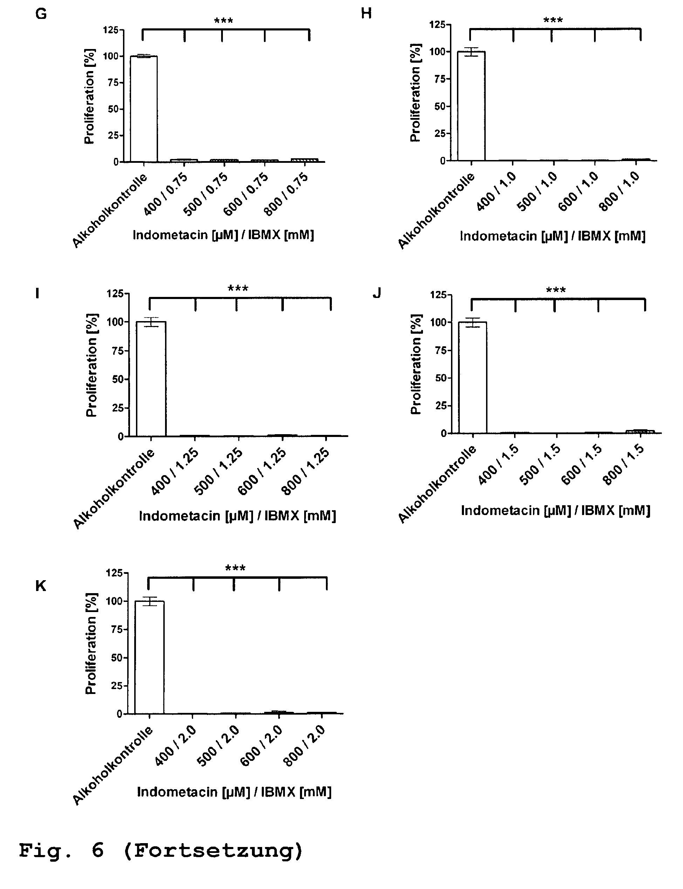

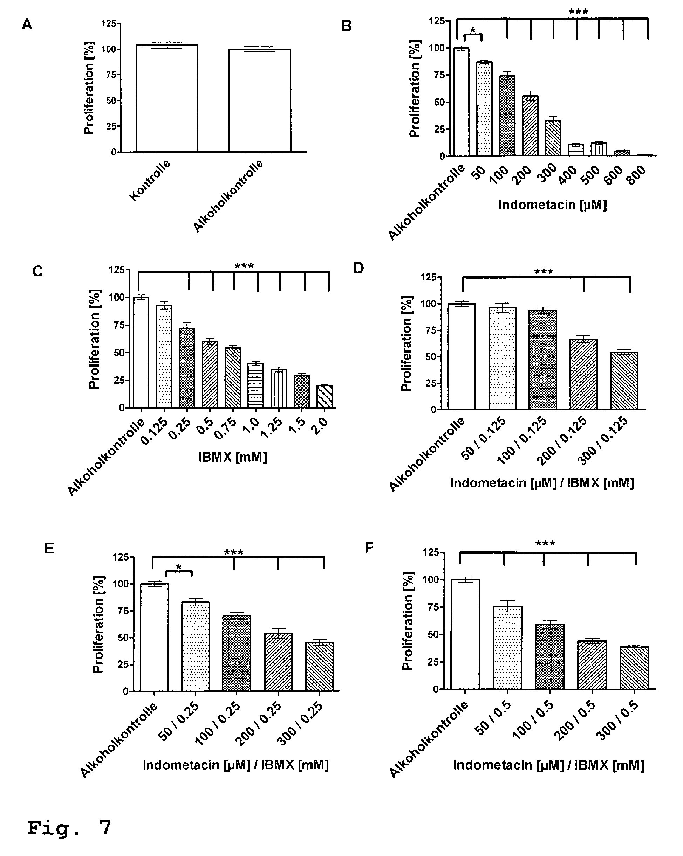

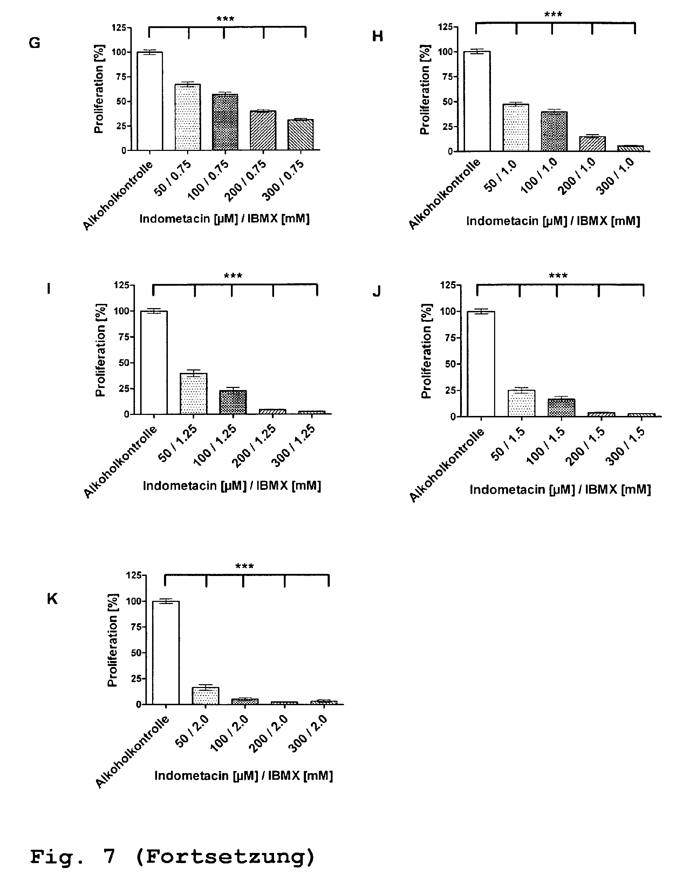

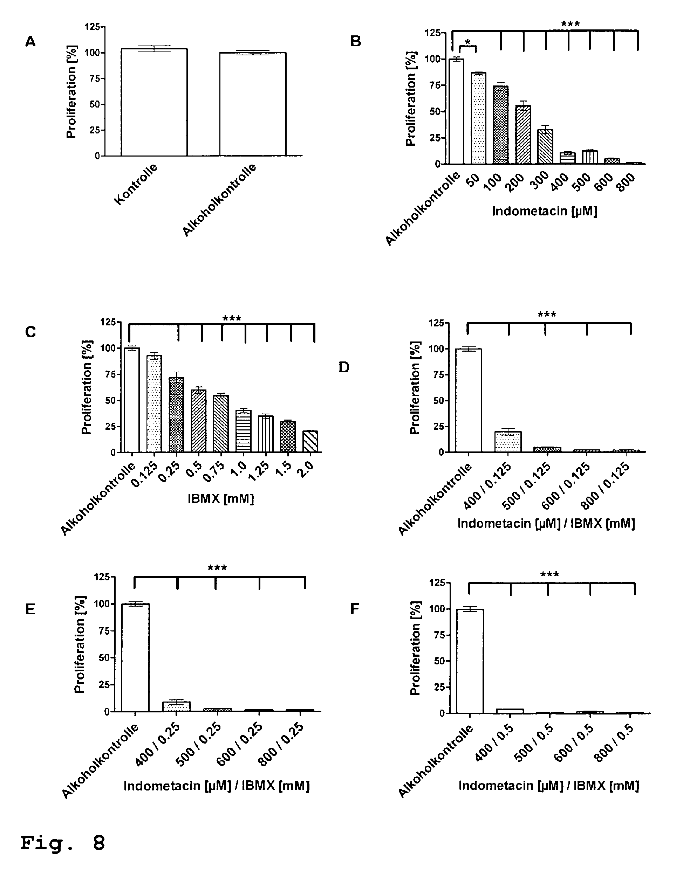

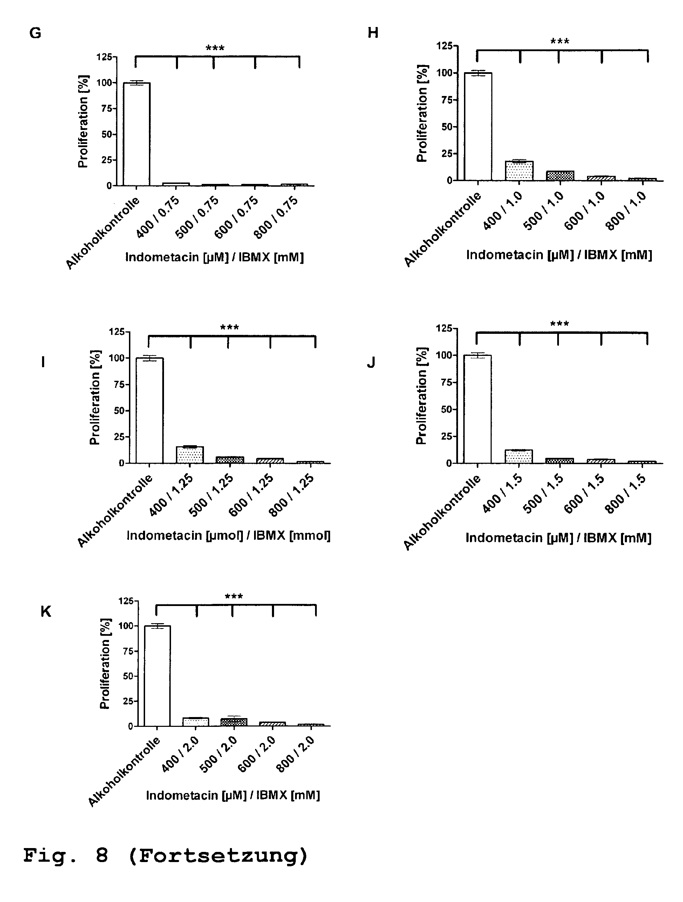

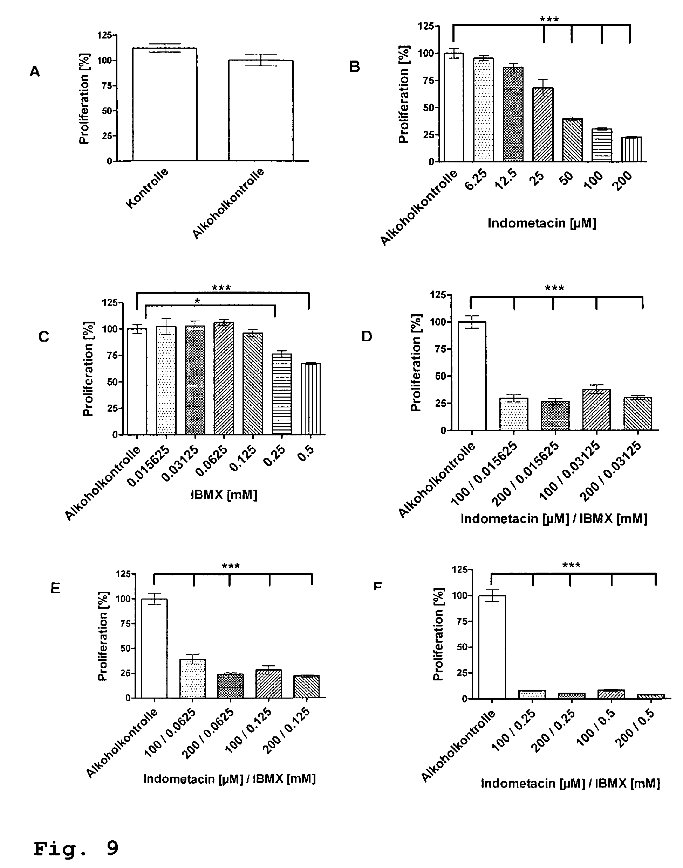

Wie in den Figuren 2-9 gezeigt, bewirkte die kombinierte Behandlung mit IBMX und Indometacin eine signifikante, synergistische Hemmung der Proliferation.

### Beispiel 6: In vitro Versuche mit Kombinationen aus Sildenafil und Meloxicam

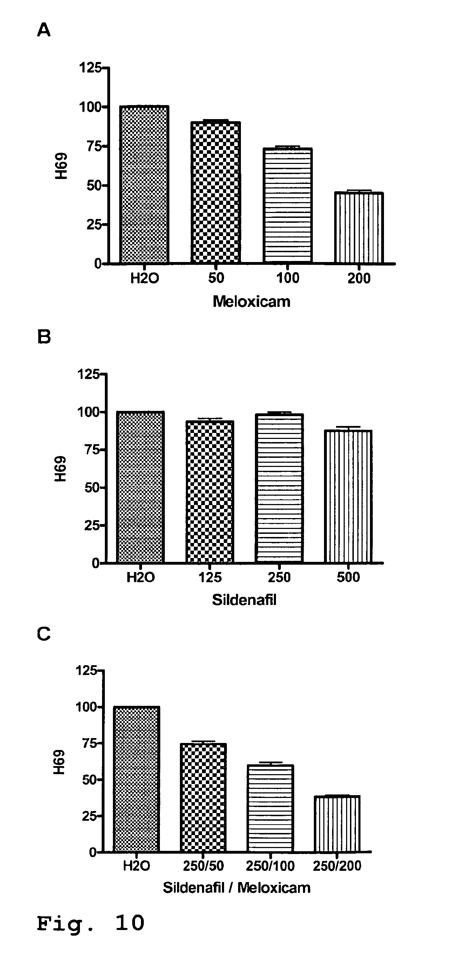

H69 Zellen wurden wie in Beispiel 5 beschrieben mit Meloxicam und Sildefanil behandelt. Meloxicam wirkt bei H69-Zellen in den gleichen Dosierungen wie Indomethacin. Die Ergebnisse sind in Figur 10 dargestellt. Auch hier lässt sich eine signifikant synergistische Wirkung auf die Proliferation der H69-Zellen beobachten.

## Patentansprüche

1. Pharmazeutische Zusammensetzung, umfassend:
a) mindestens einen Phosphodiesterasehemmer, und
b) mindestens einen COX-Inhibitor.

2. Pharmazeutische Zusammensetzung gemäß Anspruch 1 für die Behandlung einer Krebserkrankung.

3. Pharmazeutisches Kombinationspräparat, umfassend:
a) mindestens einen Phosphodiesterasehemmer, und
b) mindestens einen COX-Inhibitor,
für die gleichzeitige oder zeitlich getrennte Anwendung in der Therapie.

4. Pharmazeutisches Kombinationspräparat gemäß Anspruch 3, **dadurch gekennzeichnet, dass** der Phosphodiesterasehemmer und der COX-Inhibitor für die getrennte Verabreichung formuliert sind:

5. Pharmazeutisches Kombinationspräparat gemäß Anspruch 4, **dadurch gekennzeichnet, dass** der Phosphodiesterasehemmer und der COX-Inhibitor in unterschiedlichen Darreichungsformen formuliert sind.

6. Zusammensetzung gemäß Anspruch 1-2 oder Kombinationspräparat gemäß den Ansprüchen 3-5, **dadurch gekennzeichnet, dass** der Phosphodiesterasehemmer ausgewählt ist aus der Gruppe bestehend aus 1-Methyl-3-isobutylxanthin (IBMX), Sildenafil, Theophyllin, CC-8062, CC-8075, Vinpocetin, Anagrelid, EHNA (Erythro-9-(2-hydroxy-3-nonyl)adenin), Milrinon, Enoximon, Mesembrin, Rolipram, Ibudilast, Piclamilast, Pentoxyfyllin, Tadalafil, Vardenafil, Udenafil, Avanafil, Dipyridamol sowie pharmazeutisch geeigneten Derivaten, Salzen oder Solvaten derselben.

7. Zusammensetzung oder Kombinationspräparat gemäß Anspruch 6, **dadurch gekennzeichnet, dass** der Phosphodiesterasehemmer 1-Methyl-3-isobutylxanthin oder Sildenafil oder ein pharmazeutisch geeignetes Derivat, Salz oder Solvat davon ist.

8. Zusammensetzung gemäß Anspruch 1-2 oder Kombinatiönspräparat gemäß den Ansprüchen 3-5, **dadurch gekennzeichnet, dass** der COX-Inhibitor ausgewählt ist aus der Gruppe bestehend aus Indometacin, Meloxicam, Valdecoxib, Celecoxib, Rofecoxib, Aspirin, Ibuprofen, NS-398, Nimesulid sowie pharmazeutisch geeigneten Derivaten, Salzen oder Solvaten derselben.

9. Zusammensetzung oder Kombinationspräparat gemäß Anspruch 8, **dadurch gekennzeichnet, dass** der COX-Inhibitor Indometacin oder Meloxicam oder ein pharmazeutisch geeignetes Derivat, Salz oder Solvat davon ist.

10. Zusammensetzung oder Kombinationspräparat gemäß einem der vorhergehenden Ansprüche, **dadurch gekennzeichnet, dass** der COX-Inhibitor Indometacin oder ein pharmazeutisch geeignetes Derivat, Salz oder Solvat davon ist und der Phosphodiesterasehemmer 1-Methyl-3-isobutylxanthin oder ein pharmazeutisch geeignetes Derivat, Salz oder Solvat davon ist.

11. Verwendung eines Phosphodiesterasehemmers zur Herstellung einer pharmazeutischen Zusammensetzung für die Behandlung einer Krebserkrankung, wobei die Zusammensetzung mindestens einen Phosphodiesterasehemmer und mindestens einen COX-Inhibitor umfasst.

12. Verwendung von COX-Inhibitor zur Herstellung einer pharmazeutischen Zusammensetzung für die Behandlung einer Krebserkrankung, wobei die Zusammensetzung mindestens einen Phosphodiesterasehemmer und mindestens einen COX-Inhibitor umfasst.

13. Verwendung gemäß einem der Ansprüche 11 oder 12, **dadurch gekennzeichnet, dass** der Phosphodiesterasehemmer ausgewählt ist aus der Gruppe bestehend aus 1-Methyl-3-isobutylxanthin (IBMX), Theophyllin, CC-8062, CC-8075, Vinpocetin, Anagrelid, EHNA (Erythro-9-(2-hydroxy-3-nonyl)adenin), Milrinon, Enoximon, Mesembrin, Rolipram, Ibudilast, Piclamilast, Pentoxyfyllin, Sildenafil, Tadalafil, Vardenafil, Udenafil, Avanafil, Dipyridamol sowie pharmazeutische geeigneten Derivaten, Salzen oder Solvaten derselben.

14. Verwendung gemäß Anspruch 13, **dadurch gekennzeichnet, dass** der Phosphodiesterasehemmer 1-Methyl-3-isobutylxanthin oder Sildenafil oder ein pharmazeutisch geeignetes Derivat, Salz oder Solvat davon ist.

15. Verwendung gemäß einem der Ansprüche 11 oder 12, **dadurch gekennzeichnet, dass** der COX-Inhibitor ausgewählt ist aus der Gruppe bestehend aus Indometacin, Meloxicam, Valdecoxib, Celecoxib, Rofecoxib, Aspirin, Ibuprofen, NS-398, Nimesulid sowie pharmazeutisch geeigneten Derivaten, Salzen oder Solvaten derselben.

16. Verwendung gemäß Anspruch 15, **dadurch gekennzeichnet, dass** der COX-Inhibitor Indometacin oder Meloxicam oder ein pharmazeutisch geeignetes Derivat, Salz oder Solvat davon ist.

17. Kit, umfassend a) mindestens einen Phosphodiesterasehemmer, und b) mindestens einen COX-Inhibitor.
